# EUROPEAN PATENT SPECIFICATION

(11) **EP 0 993 437 B1**
(45) Date of publication and mention of the grant of the patent: **08.11.2006**
(21) Application number: 98932829.9
(22) Date of filing: 24.06.1998
(51) Int. Cl.: C07C 229/58, C07C 237/30, C07C 215/68, C07C 243/38, C07D 257/04, C07D 295/12, A61K 31/195

(54) **2-(4-BROMO OR 4-IODO PHENYLAMINO) BENZOIC ACID DERIVATIVES AND THEIR USE AS MEK INHIBITORS**
2-(4-BROM OR 4-IOD PHENYLAMINO)BENZOESÄUREDERIVATE UND IHRE ANWENDUNG ALS MEK-INHIBITOREN
DERIVES D'ACIDE BENZOIQUE 2-(4-BROMO OU 4-IODO PHENYLAMINO) ET UTILISATION DE CES DERNIERS EN TANT QU'INHIBITEURS DE MEK

(30) Priority: 01.07.1997 US 51433 P
(43) Date of publication of application: 19.04.2000
(73) Proprietor: Warner-Lambert Company LLC, Morris Plains, NJ 07950 (US)
(72) Inventor: BARRETT, Stephen, Douglas, Livonia, MI 48154 (US); BRIDGES, Alexander, James, Saline, MI 48176 (US); CODY, Donna, Reynolds, Saline, MI 48176 (US); DOHERTY, Annette, Marian, F-75016 Paris (FR); DUDLEY, David, Thomas, Ann Arbor, MI 48108 (US); SALTIEL, Alan, Robert, Ann Arbor, MI 48105 (US); SCHROEDER, Mel, Conrad, Dexter, MI 48130 (US); TECLE, Haile, Ann Arbor, MI 48108 (US)
(74) Representative: Dekker, Henrike Cornelie Christine
(86) International application number: PCT/US1998/013105
(87) International publication number: WO 1999/001421

(56) References cited:
- WO-A-98/37881
- US-A- 2 077 249
- US-A- 5 525 625
- BEKEMEIER H ET AL: "STRUCTURE-ACTIVITY RELATIONSHIP IN NONSTEROIDAL ANTIINFLAMMATORY AGENTS, INCLUDING OSAR IN FENAMATE DERIVATIVES" AGENTS AND ACTIONS SUPPLEMENTS, 1 July 1982, pages 17-34, XP002063635
- BERNER N H ET AL: "SUBSTITUTED N-PHENYLANTHRANILIC ACID HYDRAZIDES AS POTENTIAL ANTIMALARIAL AND ANTIMICROBIAL AGENTS" JOURNAL OF MEDICINAL CHEMISTRY, vol. 13, no. 3, 1970, pages 552-554, XP002063637
- R. M. ACHESON ET AL.: "The Condensation of Acridone with Tertiary Aromatic Amines and the Ultraviolet Absorption Spectra of the Products" JOURNAL OF THE CHEMICAL SOCIETY.,1956, pages 484-489, XP002081068 LETCHWORTH GB
- RAMANUJAM P ET AL: "ANTIFUNGAL ACTIVITY OF SOME N-SUBSTITUTED ANTHRANILIC ACID DERIVATIVES" PLANTA MEDICA, vol. 25, no. 1, 1974, pages 43-46, XP002063636
- CHEMICAL ABSTRACTS, vol. 99, no. 19, 7 November 1983 Columbus, Ohio, US; abstract no. 158369f, M. KHALIFA ET AL.: "Synthesis and Biological Activity of Certain Derivatives of 2,4-Dioxo-1,2,3,4-tetrahydroquinazoline" page 613; column 2; XP002081073 & EGYPT. J. CHEM., vol. 25, no. 3, 1983, pages 285-291,
- CHEMICAL ABSTRACTS, vol. 77, no. 19, 6 November 1972 Columbus, Ohio, US; abstract no. 122522g, I. S. SHUL'GA ET AL.: "Synthesis of 6-Nitrodiphenylamine-2-carboxylic acid Derivatives, their Physiochemical and Antimicrobial Properties" page 65; column 1; XP002081074 & FARM. ZH., vol. 27, no. 3, 1972, pages 84-85, kiev
- CHEMICAL ABSTRACTS, vol. 114, no. 19, 13 May 1991 Columbus, Ohio, US; abstract no. 184706m, A. N. GAIDUKEVICH ET AL.: "Reactivity of Derivatives of Phenylanthranilic Acid. VII. Acid-base Properties of Derivatives of Phenylanthranilic Acid in Binary Solvent Dioxane-Water" page 714; column 1; XP002081075 -& CHEMICAL ABSTRACTS 12TH COLLECTIVE INDEX., vol. 106-115, 1987 - 1991, page 14641cs XP002081069 COLUMBUS US & ORG. REACT. TARTU), vol. 27, no. 1-2, 1990, pages 87-96,
- CHEMICAL ABSTRACTS, vol. 109, no. 17, 24 October 1988 Columbus, Ohio, US; abstract no. 149000b, I. S. SHUL'GA ET AL.: "Synthesie and Phisiochemical and Biological Properties on Diphenylamine-2-carboxylic Acid Derivatives" page 682; column 1; XP002081076 -& CHEMICAL ABSTRACTS 12TH COLLECTIVE INDEX., vol. 106-115, 1987 - 1991, page 15109cs XP002081070 COLUMBUS US & FARM. ZH. (KIEV), no. 1, 1988, pages 42-45,
- CHEMICAL ABSTRACTS, vol. 101, no. 3, 16 July 1984 Columbus, Ohio, US; abstract no. 22893z, V. I. MAKURINA ET AL.: "A Study of Acid-base Equilibria in Aqueous Dioxane Solutions of Phenanthranyl Acid Derivatives" page 549; column 2; XP002081077 -& CHEMICAL ABSTRACTS 11TH COLLECTIVE INDEX., vol. 96-105, 1982 - 1986, page 10688cs XP002081071 COLUMBUS US & ORG. REACT (TARTU), vol. 20, no. 3, 1983, pages 311-315,
- CHEMICAL ABSTRACTS, vol. 83, no. 3, 21 July 1975 Columbus, Ohio, US; abstract no. 27831x, JACEK ROMANOWSKI ET AL.: "Use of 2,6-Dichlorobenzonitrile (Dichlobenil) as a Starting Substance for Synthesizing Herbicides Derived from Benzoic Acid" page 473; column 1; XP002081078 -& CHEMICAL ABSTRACTS 9TH COLLECTIVE INDEX., vol. 76-85, 1972 - 1976, page 6385cs XP002081072 COLUMBUS US & PRZEM. CHEM., vol. 54, no. 1, 1975, pages 26-31, warsaw, poland

## Description

### FIELD OF THE INVENTION

This invention provides benzoic acid and amide derivatives of anthranilic acids which inhibit certain dual specificity kinase enzymes involved in proliferative diseases such as cancer and restenosis.

### BACKGROUND OF THE INVENTION

Proliferative diseases are caused by a defect in the intracellular signaling system, or the signal transduction mechanism of certain proteins. Cancer, for example, is commonly caused by a series of defects in these signaling proteins, resulting from a change either in their intrinsic activity or in their cellular concentrations. The cell may produce a growth factor that binds to its own receptors, resulting in an autocrine loop, which continually stimulates proliferation. Mutations or overexpression of intracellular signaling proteins can lead to spurious mitogenic signals within the cell. Some of the most common mutations occur in genes encoding the protein known as Ras, which is a G-protein that is activated when bound to GTP, and inactivated when bound to GDP.

The above mentioned growth factor receptors, and many other mitogenic receptors, when activated, lead to Ras being converted from the GDP-bound state to the GTP-bound state. This signal is an absolute prerequisite for proliferation in most cell types. Defects in this signaling system, especially in the deactivation of the Ras.GTP complex, are common in cancers, and lead to the signaling cascade below Ras being chronically activated.

Activated Ras leads in turn to the activation of a cascade of serine/threonine kinases. One of the groups of kinases known to require an active Ras.GTP for its own activation is the Raf family. These in turn activate MEK, (eg, MEK₁ and MEK₂) which then activates MAP kinase. Activation of MAP kinase by mitogens appears to be essential for proliferation, and constitutive activation of this kinase is sufficient to induce cellular transformation. Blockade of downstream Ras signaling, for example by use of a dominant negative Raf-1 protein, can completely inhibit mitogenesis, whether induced from cell surface receptors or from oncogenic Ras mutants. Although Ras is not itself a protein kinase, it participates in the activation of Raf and other kinases, most likely through a phosphorylation mechanism. Once activated, Raf and other kinases phosphorylate MEK on two closely adjacent serine residues, S²¹⁸ and S²²² in the case of MEK-1, which are the prerequisite for activation of MEK as a kinase. MEK in turn phosphorylates MAP kinase on both a tyrosine, Y¹⁸⁵, and a threonine residue, T¹⁸³, separated by a single amino acid. This double phosphorylation activates MAP kinase at least 100-fold, and it can now catalyze the phosphorylation of a large number of proteins, including several transcription factors and other kinases. Many of these MAP kinase phosphorylations are mitogenically activating for the target protein, whether it be another kinase, a transcription factor, or other cellular protein. MEK is also activated by several kinases other than Raf-1, including MEKK, and itself appears to be a signal integrating kinase. As far as is currently known, MEK is highly specific for the phosphorylation of MAP kinase. In fact, no substrate for MEK other than MAP kinase has been demonstrated to date, and MEK does not phosphorylate peptides based on the MAP kinase phosphorylation sequence, or even phosphorylate denatured MAP kinase. MEK also appears to associate strongly with MAP kinase prior to phosphorylating it, suggesting that phosphorylation of MAP kinase by MEK may require a prior strong interaction between the two proteins. Both this requirement and the unusual specificity of MEK are suggestive that it may have enough difference in its mechanism of action to other protein kinases that selective inhibitors of MEK, possibly operating through allosteric mechanisms rather than through the usual blockade of the ATP binding site, may be found. US 5,525,625 relates to 2-(2-amino-3-methoxyphenyl)-4-oxo-4H[1]-benzopyran for treating proliferative disorders.

This invention provides compounds which are highly specific inhibitors of the kinase activity of MEK. Both in enzyme assays and whole cells, the compounds inhibit the phosphorylation of MAP kinase by MEK, thus preventing the activation of MAP kinase in cells in which the Ras cascade has been activated. The results of this enzyme inhibition include a reversal of transformed phenotype of some cell types, as measured both by the ability of the transformed cells to grow in an anchorage-independent manner and by the ability of some transformed cell lines to proliferate independently of external mitogens.

The compounds provided by this invention are 2-(phenylamino) benzoic acid, tetrazole, ester, amide, and benzyl alcohol derivatives, in which the phenyl ring is substituted at the 4-position with bromo or iodo. United States Patent No. 5,155,110 discloses a wide variety of fenamic acid derivatives, including certain 2-(phenylamino) benzoic acid derivatives, as anti-inflammatory agents. The reference fails to describe the compounds of this invention or their kinase inhibitory activity.

### SUMMARY OF THE INVENTION

This invention provides 4-bromo and 4-iodo phenylamino benzoic acid derivatives which are selective MEK kinase inhibitors and as such are useful for treating proliferative diseases such as cancer, psoriasis, and restenosis. The compounds are defined by Formula I 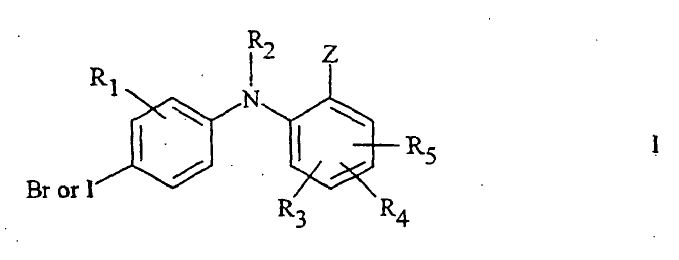 wherein:
- R₁: is hydroxy, C₁-C₈ alkyl, C₁-C₈ alkoxy, halo, trifluoromethyl, or CN;
- R₂: is hydrogen;
- R₃, R₄, and R₅: independently are hydrogen, hydroxy, halo, trifluoromethyl, C₁-C₈ alkyl, C₁-C₈ alkoxy, nitro, CN, or -(O orNH)ₘ -(CH₂)ₙ-R₉, where R₉ is hydrogen, hydroxy, CO₂H, or NR₁₀R₁₁;
- n: is 0-4;
- m: is 0 or 1;
- R₁₀ and R₁₁: independently are hydrogen or C₁-C₈ alkyl, or taken together with the nitrogen to which they are attached, can complete a 3-10 member cyclic ring optionally containing one, two, or three additional heteroatoms selected from O, S, NH, or N-C₁-C₈ alkyl;
- Z: is COOR₇, tetrazolyl, CONR₆R₇, CONHNR₁₀R₁₁, or CH₂OR₇;
- R₆ and R₇: independently are hydrogen, C₁-C₈ alkyl, C₂-C₈ alkenyl, C₂-C₈ alkynyl, 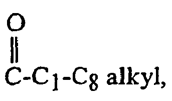 aryl, heteroaryl, C₃-C₁₀ cycloalkyl, or C₃-C₁₀ (cycloalkyl optionally containing one, two, or three heteroatoms selected from.0, S, NH, or N alkyl); or R₆ and R₇ together with the nitrogen to which they are attached complete a 3-10 member cyclic ring optionally containing 1, 2, or 3 additional heteroatoms selected from O, S, NH, or N alkyl;
and wherein any of the foregoing alkyl, alkenyl, and alkynyl groups can be unsubstituted or substituted by halo, hydroxy, alkoxy, amino, alkylamino, dialkylamino, cycloalkyl, aryl, aryloxy, heteroaryl, or heteroaryloxy, and the pharmaceutically acceptable salts thereof, with the proviso that compounds of formula I are not 2'-4-4'-tribromodiphenylamine-2-carboxylic acid, methyl ester of 2'-4-4'-tribromodiphenylamine-2-carboxylic acid or 2[(2,4-dibromophenyl)amino]-5-bromo-benzoic acid.

Preferred compounds have Formula II 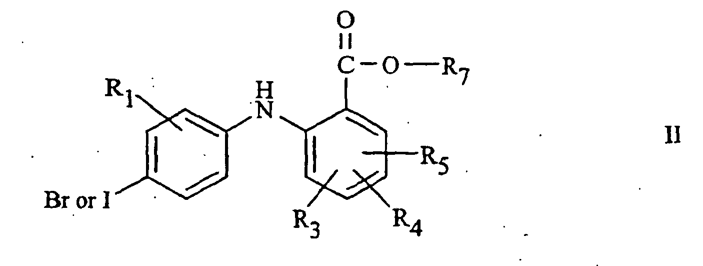 where R₁, R₃, R₄, R₅, R₆, and R₇ are as defined above. Especially preferred are compounds wherein R₁ is methyl or halo, and R₃, R₄, and R₅ are halo such as fluoro or bromo.

The compounds of Formula II are carboxylic acids when R₇ is hydrogen, and are esters when R₇ is other than hydrogen. Compounds which are analogous to the acids in physical and biological properties are tetrazolyl derivatives of Formula IIa

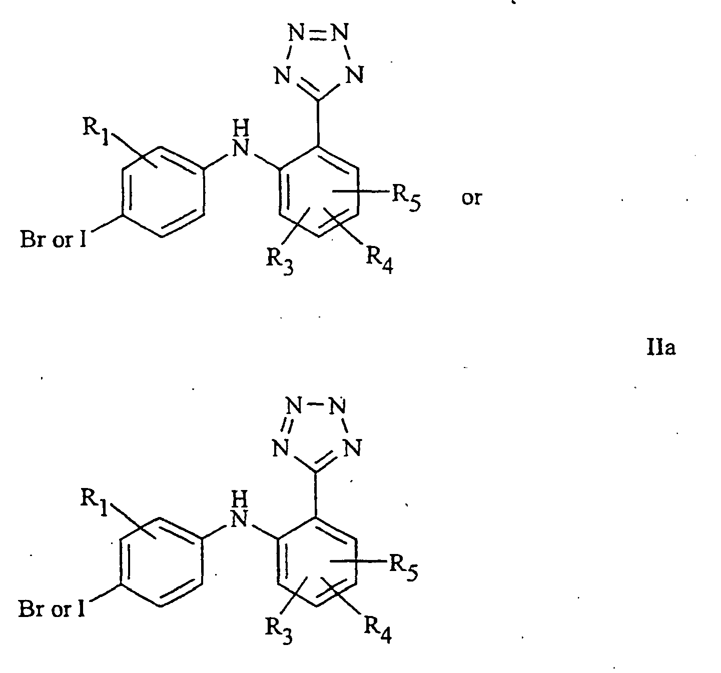

Another preferred group of compounds are amides Formula III 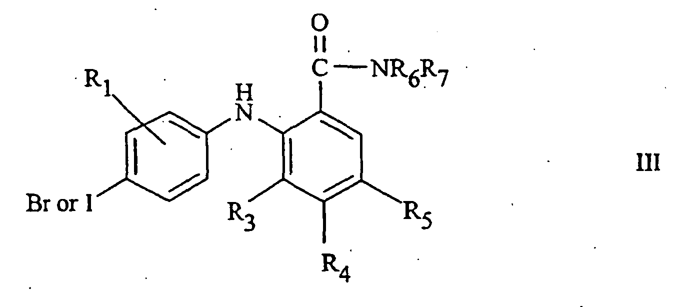 and hydrazides of Formula IIIa 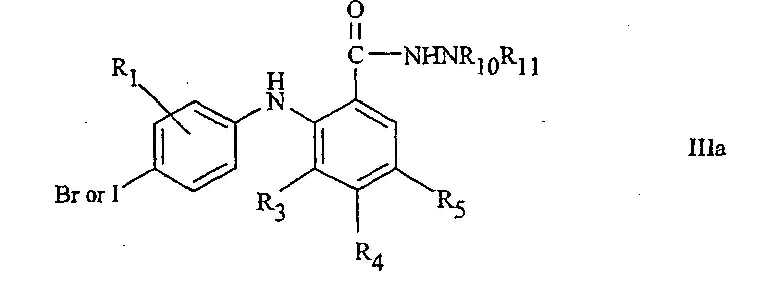 The benzyl alcohols of the invention have Formula IV

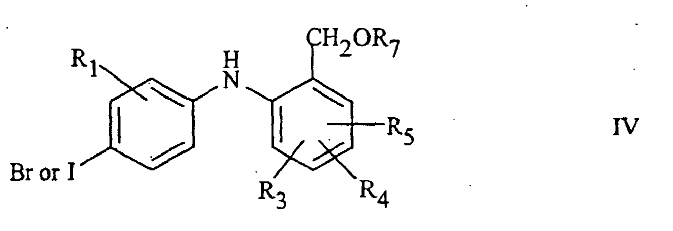

The most preferred compounds are those wherein R₁ is methyl, R₃ is hydrogen or halo such as fluoro, R₄ is halo such as fluoro, and R₅ is hydrogen or halo such as fluoro, bromo, or chloro. Representative compounds have the formulas

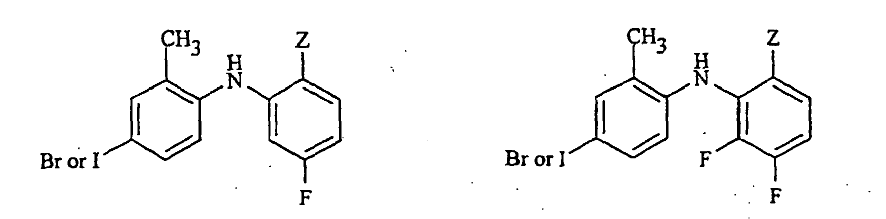

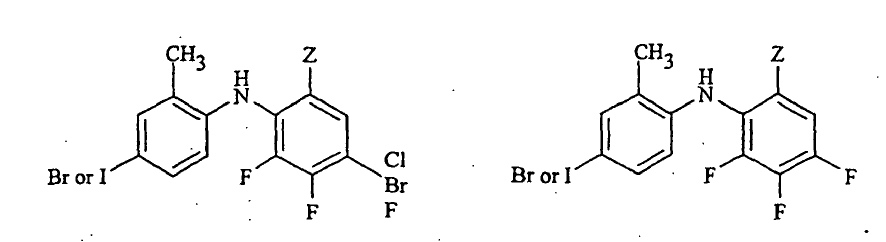

This invention also provides pharmaceutical formulations comprising a compound of Formula I together with a pharmaceutically acceptable excipient, diluent, or carrier. Preferred formulations include any of the foregoing preferred compounds together with an excipient, diluent, or carrier.

The compounds of Formula I are potent and selective inhibitors of MEK₁ and MEK₂ kinase enzymes. They are, therefore, useful to treat subjects suffering from cancer, stroke, diabetes, Alzheimer's disease, cystic fibrosis, viral disease, heart failure, and proliferative diseases such as psoriasis, restenosis, autoimmune disease, and atherosclerosis. The compounds are especially well suited to treat cancers such as breast cancer, colon cancer, prostate cancer, skin cancer, and pancreatic cancer. They are particularly well-suited for use in conjunction with conventional radiation therapy. The compounds are also immunomodulatory agents and can be used to treat degenerative diseases where change in MEK activation leads to pathologies such as hepatomegaly and cardiomegaly. The invention provides a method of inhibiting MEK enzymes and use of the compounds below for preparing a pharmaceutical composition for treating the foregoing diseases by administering to a subject an effective amount of a compound of Formula I.

### DETAILED DESCRIPTION OF THE INVENTION

As used herein, the term "aryl" means a cyclic, bicyclic, or tricyclic aromatic ring moiety having from five to twelve carbon atoms. Examples of typical aryl groups include phenyl, naphthyl, and fluorenyl. The aryl may be substituted by one, two, or three groups selected from fluoro, chloro, bromo, iodo, alkyl, hydroxy, alkoxy, nitro, amino, alkylamino, or dialkylamino. Typical substituted aryl groups include 3-fluorophenyl, 3,5-dimethoxyphenyl, 4-nitronaphthyl, 2-methyl-4-chloro-7-aminofluorenyl, and the like.

The term "aryloxy" means an aryl group bonded through an oxygen atom, for example phenoxy, 3-bromophenoxy, naphthyloxy, and 4-methyl-1-fluorenyloxy.

"Heteroaryl" means a cyclic, bicyclic, or tricyclic aromatic ring moiety having from four to eleven carbon atoms and one, two, or three heteroatoms selected from O, S, or N. Examples include furyl, thienyl, pyrrolyl, pyrazolyl, imidazolyl, triazolyl, thiazolyl, oxazolyl, xanthenyl, pyronyl, indolyl, pyrimidyl, naphthyridyl, pyridyl, benzinnidazolyl, and triazinyl. The heteroaryl groups can be unsubstituted or substituted by one, two, or three groups selected from fluoro, chloro, bromo, iodo, alkyl, hydroxy, alkoxy, nitro, amino, alkylamino, or dialkylamino. Examples of substituted heteroaryl groups include chloropyranyl, methylthienyl, fluoropyridyl, amino-1,4-benzisoxazinyl, nitroisoquinolinyl, and hydroxyindolyl.

The heteroaryl groups can be bonded through oxygen to make heteroaryloxy groups, for example thienyloxy, isothiazolyloxy, benzofuranyloxy, pyridyloxy, and 4-methylisoquinolinyl'oxy.

The term "C₁-C₈ alkyl" means straight and branched chain aliphatic groups having from one to eight carbon atoms, preferably one to four. Typical C₁-C₈ alkyl groups include methyl, ethyl, isopropyl, tert.-butyl, 2,3-dimethylhexyl, and 1,1-dimethylpentyl. The alkyl groups can be unsubstituted or substituted by halo, hydroxy, alkoxy, amino, alkylamino, dialkylamino, cycloalkyl, aryl; aryloxy, heteroaryl, or heteroaryloxy, as those terms are defined herein. Typical substituted alkyl groups include chloromethyl, 3-hydroxypropyl, 2-dimethylaminobutyl, and 2-(hydroxymethylamino)ethyl. Examples of aryl and aryloxy substituted alkyl groups include phenylmethyl, 2-phenylethyl, 3-chlorophenylmethyl, 1,1-dimethyl-3-(2-nitrophenoxy)butyl, and 3,4,5-trifluoronaphthylmethyl. Examples of alkyl groups substituted by a heteroaryl or heteroaryloxy group include thienylmethyl, 2-furylethyl, 6-furyloxyoctyl, 4-methylquinolyloxymethyl, and 6-isothiazolylhexyl. Cycloalkyl substituted alkyl groups include cyclopropylmethyl, 2-cyclohexyethyl, piperidyl-2-methyl, 2-(piperidin-1-yl)-ethyl, 3-(morpholin-4-yl)propyl.

"C₂-C₈ Alkenyl" means a straight or branched carbon chain having one or more double bonds. Examples include but-2-enyl, 2-methyl-prop-2-enyl, 1,1-dimethyl-hex-4-enyl, 3-ethyl-4-methyl-pent-2-enyl, and 3-isopropyl-pent-4-enyl. The alkenyl groups can be substituted with halo, hydroxy, alkoxy, amino, alkylamino, dialkylamino, aryl, aryloxy, heteroaryl, or heteroyloxy, for example 2-bromoethenyl, 3-hydroxy-2-butenyl, 1-aminoethenyl, 3-phenylprop-2-enyl, 6-thienyl-hex-2-enyl, 2-furyloxy-but-2-enyl, and 4-naphthyloxy-hex-2-enyl.

"C₂-C₈ Alkynyl" means a straight or branched carbon chain having from two to eight carbon atoms and at least one triple bond. Typical alkynyl groups include prop-2-ynyl, 2-methyl-hex-5-ynyl, 3,4-dimethyl-hex-5-ynyl, and 2-ethyl-but-3-ynyl. The alkynyl groups can be substituted as the alkyl and alkenyl groups, for example, by aryl, aryloxy, heteroaryl, or heteroaryloxy, for example 4-(2-fluorophenyl)-but-3-ynyl, 3-methyl-5-thienylpent-4-ynyl, 3-phenoxy-hex-4-ynyl, and 2-furyloxy-3-methyl-hex-4-ynyl.

The alkenyl and alkynyl groups can have one or more double bonds or triple bonds, respectively, or a combination of double and triple bonds. For example, typical groups having both double and triple bonds include hex-2-en-4-ynyl, 3-methyl-5-phenylpent-2-en-4-ynyl, and 3-thienyloxy-hex-3-en-5-ynyl.

The term "C₃-C₁₀ cycloalkyl" means a nonaromatic ring or fused rings containing from three to ten carbon atoms. Examples include cyclopropyl, cyclobutyl, cyclopenyl, cyclooctyl, bicycloheptyl, adamantyl, and cyclohexyl. The ring can optionally contain one, two, or three heteroatoms selected from O, S, or NR₉. Such groups include tetrahydrofuryl, tetrahydropyrrolyl, octahydrobenzofuranyl, morpholinyl, piperazinyl, pyrrolidinyl, piperidinyl, octahydroindolyl, and octahydrobenzothiofuranyl. The cycloalkyl groups can be substituted with the same substituents as an alkyl and alkenyl groups, for example, halo, hydroxy, aryl, and heteroaryloxy. Examples include 3-hydroxycyclohexyl, 2-aminocyclopropyl, 2-phenylpyrrolidinyl, and 3-thienylmorpholine-1-yl.

R₆ and R₇ can be taken together with the nitrogen to which they are attached to complete a cyclic ring having from 3 to 10 members, which may contain 1,2, or 3 additional heteroatoms selected from O, S, NH, or N alkyl. Examples of such cyclic rings include piperazinyl, piperidyl, pyrrolidinyl, morpholino, N-methylpiperazinyl, aziridynyl, and the like. Such rings can be substituted with halo, hydroxy, alkyl, alkoxy, amino, alkyl, and dialkylamino, aryl, aryloxy, heteroaryl, and heteroaryloxy. Typical examples include 3-hydroxy-pyrrolidinyl, 2-fluoro-piperindyl, 4-(2-hydroxyethyl)-piperidinyl, and 3-thienylmorpholino.

The 2-(4-bromo and 4-iodo phenylamino)-benzoic acid derivatives of Formula I can be prepared from commercially available starting materials utilizing synthetic methodologies well-known to those skilled in organic chemistry. A typical synthesis is carried out by reacting a 4-bromo or 4-iodo aniline with a benzoic acid having a leaving group at the 2-position to give a 2-(phenylamino)-benzoic acid. This process is depicted in Scheme 1. 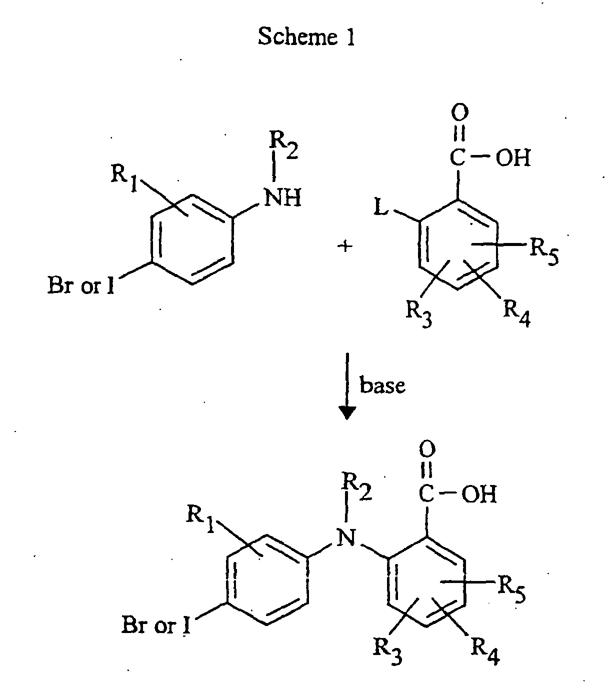 where L is a leaving group, for example halo such as fluoro.

The reaction of aniline and the benzoic acid derivative generally is accomplished by mixing the benzoic acid with an equimolar quantity or excess of the aniline in an unreactive organic solvent such as tetrahydrofuran or toluene, in the presence of a base such as lithium diisopropylamide, n-butyl lithium, sodium hydride, triethylamine, and Hunig's base. The reaction generally is carried out at a temperature of about -78°C to about 100°C, and normally is complete within about 2 hours to about 4 days. The product can be isolated by removing the solvent, for example by evaporation under reduced pressure, and further purified, if desired, by standard methods such as chromatography, crystallization, or distillation.

The 2-(phenylamino)-benzoic acid (eg, Formula I, where R₇ is hydrogen) can be reacted with an organic or inorganic base such as pyridine, triethylamine, calcium carbonate, or sodium hydroxide to produce a pharmaceutically acceptable salt. The free acids can also be reacted with an alcohol of the formula HOR₇ (where R₇ is other than hydrogen, for example methyl) to produce the corresponding ester. Reaction of the benzoic acid with an alcohol can be carried out in the presence of a coupling agent. Typical coupling reagents include 2-ethoxy-1-ethoxycarbonyl-1,2-dihydroquinoline (EEDQ), 1,3-dicyclohexylcarbodiimide (DCC), bromo-tris(pyrrolidino)-phosphonium hexafluorophosphate (PyBrOP), and (benzotriazolyloxy) tripyrrolidino phosphonium hexafluorophosphate (PyBOP). The phenylamino benzoic acid and alcohol derivative normally are mixed in approximately equimolar quantities in an unreactive organic solvent such as dichloromethane, tetrahydrofuran, chloroform, or xylene, and an equimolar quantity of the coupling reagent is added. A base such as triethylamine or diisopropylethylamine can be added to act as an acid scavenger if desired. The coupling reaction generally is complete after about 10 minutes to 2 hours, and the product is readily isolated by removing the reaction solvent, for instance by evaporation under reduced pressure, and purifying the product by standard methods such as chromatography or crystallizations from solvents such as acetone, diethyl ether, or ethanol.

The benzamides of the invention, Formula I where Z is CONR₆R₇, are readily prepared by reacting the foregoing benzoic acids with an amine of the formula HNR₆R₇. The reaction is carried out by reacting approximately equimolar quantities of the benzoic acid and amine in an unreactive organic solvent in the presence of a coupling reagent. Typical solvents are chloroform, dichloromethane, tetrahydrofuran, benzene, toluene, and xylene. Typical coupling reagents include DCC, EEDQ, PyBrOP, and PyBOP. The reaction is generally complete after about 10 minutes to about 2 hours when carried out at a temperature of about 0°C to about 60°C. The product amide is readily isolated by removing the reaction solvent, for instance by evaporation, and further purification can be accomplished by normal methods such as chromatography, crystallization, or distillation. The hydrazides (z = CONHNR₁₀R₁₁) are similarly prepared by coupling a benzoic acid with a hydrazine of the formula H₂HNR₁₀R₁₁.

The benzyl alcohols of the invention, compounds of Formula I where Z is CH₂OR₆ and R₆ is hydrogen, are readily prepared by reduction of the corresponding benzoic acid according to the following scheme 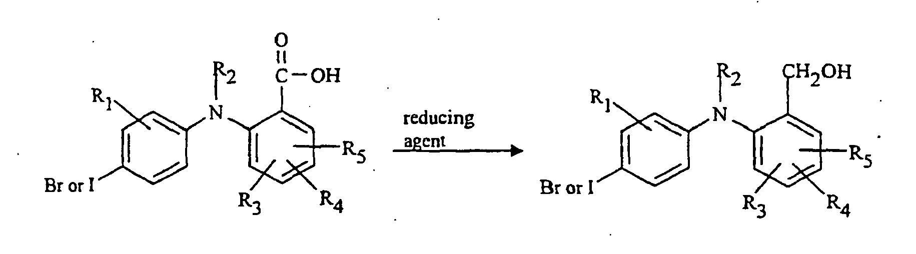 Typical reducing agents commonly employed include borane in tetrahydrofuran. The reduction normally is carried out in an unreactive organic solvent such as tetrahydrofuran, and generally is complete within about 2 hours to about 24 hours when conducted at a temperature of about 0°C to about 40°C.

The following detailed examples illustrate specific compounds provided by this invention.

### EXAMPLE 1

### 4-Fluoro-2-(4-iodo-2-methylphenylamino)benzoic acid

To a stirring solution comprised of 3.16 g (0.0133 mol) of 2-amino-5-iodotoluene in 5 mL of tetrahydrofuran at -78°C was added 10 mL (0.020 mol) of a 2.0 M lithium diisopropylamide in tetrahydrofuran/heptane/ethenylbenzene (Aldrich) solution. The resulting green suspension was stirred vigorously for 15 minutes, after which time a solution of 1.00 g (0.00632 mol) of 2,4-difluorobenzoic acid in 10 mL of tetrahydrofuran was added. The reaction temperature was allowed to increase slowly to room temperature, at which temperature it was stirred for 2 days. The reaction mixture was concentrated. Aqueous HCl (10%) was added to the concentrate, and the solution was extracted with dichloromethane. The organic phase was dried (MgSO₄) and then boiled over a steambath to low volume and cooled to room temperature. The off-white fibers were collected by vacuum filtration, rinsed with hexanes, and vacuum-oven dried. (76°C; ca. 10 mm of Hg) to afford 1.10 g (47%) of the desired material; mp 224-229.5°C;
¹H NMR (400 MHz; DMSO): Λ 9.72 (s, 1H), 7.97 (dd, 1H, J = 7.0, 8.7 Hz), 7.70 (d, 1H, J = 1.5 Hz), 7.57 (dd, 1H, J = 8.4, 1.9 Hz), 7.17 (d, 1H, J = 8.2 Hz), 6.61-6.53 (m, 2H), 2.18 (s, 3H);
13C NMR (100 MHz; DMSO): Λ 169.87, 167.60, 165.12, 150.17, 150.05, 139.83, 138.49, 136.07, 135.31, 135.20, 135.07, 125.60, 109.32, 105.09, 104.87, 99.72, 99.46, 89.43, 17.52;
¹⁹F NMR (376 MHz; DMSO): δ-104.00 to -104.07 (m);
IR (KBr) 1670 (C = O stretch) cm⁻¹;
MS (CI) M+1 = 372.

| Analysis calculated for C₁₄H₁₁FINO₂: | | | |
|---|---|---|---|
| | C, 45.31; | H, 2.99; | N, 3.77. |
| Found: | C, 45.21; | H, 2.77; | N, 3.64. |

### EXAMPLES 2-30

By following the general procedure of Example 1, the following benzoic acids and salts were prepared:

| Example No. | Compound | MP °C |
|---|---|---|
| 2 | 3,4,5-Trifluoro-2-(4-iodo-2-methyl-phenylamino)-benzoic acid | 206-210 |
| 3 | 3,4-Difluoro-2-(4-iodo-2-methyl-phenylamino)-benzoic acid | 240.5-244.5 |
| 4 | 5-Bromo-3,4-difluoro-2-(4-iodo-2-methyl-phenylamino)-benzoic acid | 259.5-262 |
| 5 | 5-Chloro-2-(2-chloro-4-iodo-phenylamino)-benzoic acid | 255-260 |
| 6 | 5-Chloro-2-(4-iodo-2-methyl-phenylamino)-benzoic acid | 234-238 |
| 7 | Sodium 5-Chloro-2-(4-iodo-2-methyl-phenylamino)-benzoate | 310-320 DEC |
| 8 | 5-Bromo-2-(4-iodo-2-methyl-phenylamino)-benzoic acid | 239.5-240 |
| 9 | 2-(2-Chloro-4-iodo-phenylamino)-5-nitro-benzoic acid | 289-293 |
| 10 | 4-Fluoro-2-(3-fluoro-4-iodo-2-methyl-phenylamino)-benzoic acid | 233-235 |
| 11 | 2-(4-Iodo-2-methyl-phenylamino)-5-nitro-benzoic acid | 264-267 |
| 12 | 2-(2-Fluoro-4-iodo-phenylamino)-5-nitro-benzoic acid | 256-258 |
| 13 | 2-(4-Bromo-2-methyl-phenylamino)-4-fluoro-benzoic acid | 218.5-220 |
| 14 | 2-(2-Bromo-4-iodo-phenylamino)-5-nitro-benzoic acid | 285-288 DEC |
| 15 | 2-(4-Bromo-2-methyl-phenylamino)-3,4-difluoro-benzoic acid | 230-234 |
| 16 | 3-Fluoro-2-(4-iodo-2-methyl-phenylamino)-benzoic acid | 218-221 |
| 17 | 3,4-Difluoro-2-(4-iodo-2-methoxy-phenylamino)-benzoic acid | 230-233 |
| 18 | 4-Chloro-2-(4-iodo-2-methyl-phenylamino)-benzoic acid | 245-255 DEC |
| 19 | 2-(4-Iodo-2-methyl-phenylamino)-benzoic acid | 218-223 |
| 20 | 5-Fluoro-2-(4-iodo-2-methyl-phenylamino)-benzoic acid | 243-46 |
| 21 | 5-Iodo-2-(4-iodo-2-methyl-phenylamino)-benzoic acid | 241-245 |
| 22 | 2,3 ,5 -Trifluoro-4-(4-iodo-2-methyl-phenylamino)-benzoic acid | 218-222 |
| 23 | 4-Fluoro-2-(3-chloro-4-iodo-2-methyl-phenylamino)-benzoic acid | 248-252.5 |
| 24 | 2-(4-Iodo-phenylamino)-5-methoxy-benzoic acid | 208-211 |
| 25 | 3-Chloro-2-(2-chloro-4-iodo-phenylamino)-benzoic acid | 232-233 |
| 26 | 2-Fluoro-6-(4-iodo-2-methyl-phenylamino)-benzoic acid | 179-182 |
| 27 | 4-Fluoro2-(2,3-dimethyl-4-iodo-2-methyl-phenylamino)-benzoic acid | 258-261 |
| 28 | 5-Methyl-2-(4-iodo-2-methyl-phenylamino)-benzoic acid | 209.5-211 |
| 29 | 2-Chloro-6-(4-iodo-2-methyl-phenylamino)-benzoic acid | 171-175 |
| 30 | 2-(4-Iodo-2-methyl-phenylamino)-4-nitro-benzoic acid | 251-263 |

### EXAMPLE 31

### 5-Chloro-N-(2-hydroxyethyl)-2-(4-iodo-2-methyl-phenylamino)-benzamide

To a stirring solution comprised of 0.1020 g (0.2632 mmol) of 5-chloro-2-(4-iodo-2-methyl-phenylamino)-benzoic acid, 0.1 mL (1.7 mmol) of ethanolamine, and 0.05 mL (0.29 mmol) of diisopropylethylamine in 5 mL of a 1:1 (v/v) tetrahydrofuran-dichloromethane solution was added 0.15 g (0.29 mmol) of solid PyBOP powder directly. The reaction mixture was stirred at room temperature overnight. The solvent was removed in vacuo. The crude residue was partitioned between ether (50 mL) and 10% aqueous hydrochloric acid (50 mL). The organic phase was washed with 10% aqueous sodium hydroxide (50 mL), dried (MgSO₄) and concentrated in vacuo to afford a yellow-brown oil which was crystallized from hexanes-ether to afford 0.0831 g (73%) of a green-yellow powder; mp 120-121°C;
¹H NMR (400 MHz; CDCl₃): δ 9,11 (s, 1H), 7.56 (d, 1H, J = 1.4 Hz), 7.46-7.4.1 (m, 2H), 7.20 (dd, 1H, J = 8.9, 2.4 Hz), 7.00 (t, 2H, J = 9.6 Hz), 6.55 (broad t, 1H), 3.86 (t, 2H, J = 5.0 Hz), 3.61 (dd, 2H, J = 10.1, 5.5 Hz), 2.23 (s, 3H), 1.56 (broad s, 1H);
IR (KBr) 3297 (O-H stretch), 1627 (C = O stretch) cm⁻¹;
MS (CI) M+1 = 431.

| Analysis calculated for C₁₆H₁₆ClIN₂O₂: | | | |
|---|---|---|---|
| | C, 44.62; | H, 3.74; | N, 6.50. |
| Found: | C, 44.63; | H, 3.67; | N, 6.30. |

### EXAMPLES 32-48

By following the general procedure of Example 31, the following benzamides were prepared by reacting the corresponding benzoic acid with the corresponding amine.

| Example No. | Compound | MP°C |
|---|---|---|
| 32 | 4-Methoxy-N-(4-methoxy-phenyl)-3-nitro-benzamide | 153.5-156 |
| 33 | 4-Fluoro-2-(4-iodo-2-methyl-phenylamino)-benzamide | 158 |
| 34 | 4-Fluoro-2-(4-iodo-2-methyl-phenylamino)-N-methyl-benzamide | 102.5-104.5 |
| 35 | N-Ethyl-4-fluoro-2-(4-iodo-2-methyl-phenylamino)-benzamide | 90-91 |
| 36 | 4-Fluoro-2-(4-iodo-2-methyl-phenylamino)-N,N-dimethyl-benzamide | oil |
| 37 | 4-Fluoro-2-(4-iodo-2-methyl-phenylamino)-N-(1H-tetrazol-5-yl)-benzamide | 285-288 DEC |
| 38 | 5-Bromo-2-(4-iodo-2-methyl-phenylamino)-benzamide | 180-182 |
| 39 | 5-Chloro-2-(4-iodo-2-methyl-phenylamino)-N,N-dimethyl-benzamide | 137-138 |
| 40 | [5-Chloro-2-(4-iodo-2-methyl-phenylamino)-N-hydroxycarbonylmethyl-benzamide | 170-173 |
| 41 | 4-Fluoro-2-(4-iodo-2-methyl-phenylamino)-N-propyl-benzamide | 69-71 |
| 42 | 5-Bromo-N-(2-hydroxy-ethyl)-2-(4-iodo-2-methyl-phenylamino)-benzamide | 132-133.4 |
| 43 | N,N-Diethyl-4-fluoro-2-(4-iodo-2-methyl-phenylamino)-benzamide | oil |
| 44 | 4-Fluoro-N-{3-[4-(2-hydroxy-ethyl)-piperazin-1-yl]-propyl}-2-(4-iodo-2-methyl-phenylamino)-benzamide | 122-124 |
| 45 | N,N-Diethyl-2-(4-iodo-2-methyl-phenylamino)-5-nitro-benzamide | 91-93 |
| 46 | N-Butyl-4-fluoro-2-(4-iodo-2-methyl-phenylamino)-benzamide | 97-99 |
| 47 | 5-Chloro-N,N-diethyl-2-(4-iodo-2-methyl-phenylamino)-benzamide | 118-120 |
| 48 | 5-Bromo-2-(4-iodo-2-methyl-phenylamino)-N,N-dimethyl-benzamide | 142.5-144 |

### EXAMPLE 49

### 4-Fluoro-2-(4-iodo-2-methyl-phenylamino)-benzyl alcohol

4-Fluoro-2-(4-iodo-2-methyl-phenylamino)-benzoic acid (0.50 g, 1.35 mmol) was dissolved in 6 mL (6 mmol) of cold 1.0 M boranetetrahydrofuran complex in tetrahydrofuran solution. The reaction mixture was stirred under nitrogen atmosphere at room temperature overnight. The reaction was quenched with 80 mL of methanol. Concentration in vacuo produced a clear tan oil which was purified by MPLC. Elution with dichloromethane afforded 0.4285 g (89%) of a white solid; mp 99-100.5°C;
¹H NMR (400 MHz; DMSO): δ 7.57 (d, 1H, J=1.7 Hz), 7.45 (dd, 1H, J=8.4, 1.9 Hz), 7.39 (s, 1H), 7.29 (t, 1H, J=7.5 Hz), 6.89 (d, 1H, J=8.4 Hz), 6.67-6.60 (m, 1H), 5.47 (t, 1H, J=5.5 Hz), 4.49 (d, 2H, 5.1 Hz), 2.14 (s, 3H);
IR (KBr) 3372 (O-H stretch) cm⁻¹;
MS (CI) M+1 = 358.

| Analysis calculated for C₁₄H₁₃FINO: | | | |
|---|---|---|---|
| | C, 47.08; | H, 3.67; | N, 3.92. |
| Found: | C, 47.17; | H, 3.75; | N, 3.72. |

### EXAMPLE 50-52

The following benzyl alcohols were prepared by the general procedure of Example 49.

| Example No. | Compound | MP °C |
|---|---|---|
| 50 | [5-Chloro-2-(4-iodo-2-methyl-phenylamino)-phenyl]-methanol | 82-85 |
| 51 | [2-(4-Iodo-2-methyl-phenylamino)-5-nitro-phenyl]-methanol | 126.5-128.5 |
| 52 | [5-Bromo-2-(4-iodo-2-methyl-phenylamino)-phenyl]-methanol | 60.5-63.5 |

Several invention compounds of Formula I were prepared utilizing combinatorial synthetic techniques. The general procedure is as follows:

To a 0.8-mL autosampler vial in a metal block was added 40 µL of a 0.5 M solution of the acid in DMF and 40 µL of the reagent amine (2M solution in Hunig's base and 1 M in amine in DMF). A 0.5M solution of PyBrop was freshly prepared and 50 µL were added to the autosampler vial. The reaction was allowed to stand for 24 hours.

The reaction mixture was transferred to a 2-dram vial and diluted with 2 mL of ethyl acetate. The organic layer was washed with 3 mL of distilled water and the water layer washed again with 2 mL of ethyl acetate. The combined organic layers were allowed to evaporate to dryness in an open fume hood.

The residue was taken up in 2 mL of 50% acetonitrile in water and injected on a semi-prep reversed phase column (10 mm × 25 cm, 5 µM spherical silica, pore size 115 A derivatized with C-18, the sample was eluted at 4.7 mL/min with a linear ramp to 100% acetonitrile over 8.5 minutes. Elution with 100% acetonitrile continued for 8 minutes). Fractions were collected by monitoring at 214 nM. The residue was dissolved in chloroform and transferred to a preweighed vial, evaporated, and weighed again to determine the yield.

### EXAMPLES 53-206

The following compounds of Formula I were prepared by combinatorial methodology:

| Example No. | Compound | MS M-H |
|---|---|---|
| 53 | 5-Bromo-3,4-difluoro-N-(2-hydroxy-ethyl)-2-(4-iodo-2-methyl-phenylamino)-benzamide | 510 |
| 54 | N-(2,3-Dihydroxy-propyl)-3,4-difluoro-2-(4-iodo-2-methyl-phenylamino)-benzamide | 462 |
| 55 | 5-Bromo-3,4-difluoro-2-(4-iodo-2-methyl-phenylamino)-N-(2-piperidin-1-yl-ethyl)-benzamide | 577 |
| 56 | 3,4-Difluoro-N-(2-hydroxy-ethyl)-2-(4-iodo-2-methyl-phenylamino)-benzamide | 432 |
| 57 | N-(2,3-Dihydroxy-propyl)-4-fluoro-2-(4-iodo-2-methyl-phenylamino)-benzamide | 444 |
| 58 | 3,4-Difluoro-N-(3-hydroxy-propyl)-2-(4-iodo-2-methyl-phenylamino)-benzamide | 446 |
| 59 | 5-Bromo-3,4-difluoro-2-(4-iodo-2-methyl-phenylamino)-N-(2-pyrrolidin-1-yl-ethyl)-benzamide | 564 |
| 60 | 5-Bromo-3,4-difluoro-2-(4-iodo-2-methyl-phenylamino)-N-(2-pyridin-4-yl-ethyl)-benzamide | 571 |
| 61 | 4-Fluoro-N-(2-hydroxy-ethyl)-2-(4-iodo-2-methyl-phenylamino)-benzamide | 414 |
| 62 | 5-Bromo-N-(3-dimethylamino-propyl)-3,4-difluoro-2-(4-iodo-2-methyl-phenylamino)-benzamide | 551 |
| 63 | 5-Bromo-3,4-difluoro-2-(4-iodo-2-methyl-phenylamino)-N-(2-morpholin-4-yl-ethyl)-benzamide | 580 |
| 64 | 3,4-Difluoro-2-(4-iodo-2-methyl-phenylamino)-N-(2-morpholin-4-yl-ethyl)-benzamide | 501 |
| 65 | 3,4-Difluoro-2-(4-iodo-2-methyl-phenylamino)-N-(2-pyrrolidin-1-yl-ethyl)-benzamide | 485 |
| 66 | 3,4-Difluoro-2-(4-iodo-2-methyl-phenylamino)-N-(2-pyridin-4-yl-ethyl)-benzamide | 493 |
| 67 | N-(3-Dimethylamino-propyl)-3,4-difluoro-2-(4-iodo-2-methyl-phenylamino)-benzamide | 473 |
| 68 | N-Benzyl-4-fluoro-2-(4-iodo-2-methyl-phenylamino)-benzamide | 460 |
| 69 | 2-(4-Bromo-2-methyl-phenylamino)-3,4-difluoro-N-(2-hydroxy-ethyl)-benzamide | 384 |
| 70 | 4-Fluoro-2-(4-iodo-2-methyl-phenylamino)-N-(2-morpholin-4-yl-ethyl)-benzamide | 483 |
| 71 | 4-Fluoro-2-(4-iodo-2-methyl-phenylamino)-N-(3-piperidin-1-yl-propyl)-benzamide | 495 |
| 72 | 3,4-Difluoro-2-(4-iodo-2-methyl-phenylamino)-N-(3-piperidin-1-yl-propyl)-benzamide | 513 |
| 73 | 4-Fluoro-2-(4-iodo-2-methyl-phenylamino)-N-(2-thiophen-2-yl-ethyl)-benzamide | 480 |
| 74 | 4-Fluoro-2-(4-iodo-2-methyl-phenylamino)-N-(2-pyrrolidin-1-yl-ethyl)-benzamide | 467 |
| 75 | 2-(4-Bromo-2-methyl-phenylamino)-3,4-difluoro-N-(2-morpholin-4-yl-ethyl)-benzamide | 453 |
| 76 | 5-Bromo-3,4-difluoro-2-(4-iodo-2-methyl-phenylamino)-N-pyridin-4-ylmethyl-benzamide | 557 |
| 77 | 3,4-Difluoro-2-(4-iodo-2-methyl-phenylamino)-N-pyridin-4-ylmethyl-benzamide | 479 |
| 78 | 2-(4-Bromo-2-methyl-phenylamino) N-(3-dimethylamino-propyl)-3,4-difluoro-benzamide | 425 |
| 79 | 4-Fluoro-2-(4-iodo-2-methyl-phenylamino)-N-pyridin-4-ylmethyl-benzamide | 461 |
| 80 | 4-Fluoro-2-(4-iodo-2-methyl-phenylamino)-N-(2-pyridin-4-yl-ethyl)-benzamide | 475 |
| 81 | 2-(4-Bromo-2-methyl-phenylamino)-3,4-difluoro-N-(2-pyridin-4-yl-ethyl)-benzamide | 445 |
| 82 | 2-(4-Bromo-2-methyl-phenylamino)-3,4-difluoro-N-(3-hydroxy-propyl)-benzamide | 400 |
| 83 | 2-(4-Bromo-2-methyl-phenylamino)-3,4-difluoro-N-(2-pyrrolidin-1-yl-ethyl)-benzamide | 437 |
| 84 | 4-Fluoro-2-(4-iodo-2-methyl-phenylamino)-N-phenethyl-benzamide | 474 |
| 85 | 2-(4-Bromo-2-methyl-phenylamino)-3,4-difluoro-N-(2-thiophen-2-yl-ethyl)-benzamide | 450 |
| 86 | 2-(4-Bromo-2-methyl-phenylamino)-3,4-difluoro-N-pyridin-4-ylmethyl-benzamide | 431 |
| 87 | 2-(4-Bromo-2-methyl-phenylamino)-3,4-difluoro-N-phenethyl-benzamide | 444 |
| 88 | 2-(4-Bromo-2-methyl-phenylamino)-3,4-difluoro-N-(2-piperidin-1-yl-ethyl)-benzamide | 451 |
| 89 | 5-Chloro-N-{3-[4-(2-hydroxy-ethyl)-piperazin-1-yl]-propyl}-2-(4-iodo-2-methyl-phenylamino)-benzamide | 557* |
| 90 | 5-Fluoro-N-{3-[4-(2-hydroxy-ethyl)-piperazin-1-yl]-propyl}-2-(4-iodo-2-methyl-phenylamino)-benzamide | 541* |
| 91 | 2-(4-Iodo-2-methyl-phenylamino)-5-nitro-N-pyridin-4-yl methyl-benzamide | 487 |
| 92 | 5-Bromo-N-{3-[4-(2-hydroxy-ethyl)-piperazin-1-yl]-propyl}-2-(4-iodo-2-methyl-phenylamino)-benzamide | 601* |
| 93 | 5-Chloro-N-(2-diethylamino-ethyl)-2-(4-iodo-2-methyl-phenylamino)-benzamide | 486* |
| 94 | 5-Chloro-2-(4-iodo-2-methyl-phenylamino)-N-(2-piperidin-1-yl-ethyl)-benzamide | 497* |
| 95 | (3-Hydroxy-pyrrolidin-1-yl)-[2-(4-iodo-2-methyl-phenylamino)-5-nitro-phenyl]- methanone | 466 |
| 96 | 5-Chloro-2-(4-iodo-2-methyl-phenylamino)-N-(2-pyrrolidin-1-yl-ethyl)-benzamide | 484* |
| 97 | 5-Bromo-N-(2-diethylamino-ethyl)-2-(4-iodo-2-methyl-phenylamino)-benzamide | 530* |
| 98 | N-{2-[Bis-(2-hydroxy-ethyl)-amino]-ethyl}-5-chloro-2-(4-iodo-2-methyl-phenylamino)-benzamide | 518* |
| 99 | N-{2-[Bis-(2-hydroxy-ethyl)-amino]-ethyl}-5-bromo-2-(4-iodo-2-methyl-phenylamino)-benzamide | 562* |
| 100 | [5-Bromo-2-(4-iodo-2-methyl-phenylamino)-phenyl]-(3-hydroxy-pyrrolidin-1-yl)- | 499 |
| 101 | 2-(4-Iodo-2-methyl-phenylamino)-5-nitro-benzoic acid phenethyl ester | 501 |
| 102 | N-{3-[4-(2-Hydroxy-ethyl)-piperazin-1-yl]-propyl}-2-(4-iodo-2-methyl-phenylamino)-benzamide | 568* |
| 103 | [5-Chloro-2-(4-iodo-2-methyl-phenylamino)-phenyl]-(3-hydroxy-pyrrolidin-1-yl)- | 455 |
| 104 | 5-Fluoro-2-(4-iodo-2-methyl-phenylamino)-N-pyridin-4-ylmethyl-benzamide | 460 |
| 105 | 5-Bromo-2-(4-iodo-2-methyl-phenylamino)-N-(2-pyrrolidin-1-yl-ethyl)-benzamide | 528* |
| 106 | 5-Bromo-2-(4-iodo-2-methyl-phenylamino)-N-(2-piperidin-1-yl-ethyl)-benzamide | 542* |
| 107 | 5-Fluoro-2-(4-iodo-2-methyl-phenylamino)-N-(2-pyrrolidin-1-yl-ethyl)-benzamide | 468* |
| 108 | 5-Chloro-N-(3-dimethylamino-propyl)-2-(4-iodo-2-methyl-phenylamino)-benzamide | 472* |
| 109 | N-{2-[Bis-(2-hydroxy-ethyl)-amino]-ethyl}-5-fluoro-2-(4-iodo-2-methyl-phenylamino)-benzamide . | 502* |
| 110 | 5-Chloro-N-(3-hydroxy-propyl)-2-(4-iodo-2-methyl-phenylamino)-benzamide | 445* |
| 111 | 5-Chloro-N-(3-diethylamino-2-hydroxy-propyl)-2-(4-iodo-2-methyl-phenylamino)-benzamide | 516* |
| 112 | 5-Fluoro-2-(4-iodo-2-methyl-phenylamino)-N-(2-piperidin-1-yl-ethyl)-benzamide | 482* |
| 113 | 5-Bromo-N-(3-hydroxy-propyl)-2-(4-iodo-2-methyl-phenylamino)-benzamide | 489* |
| 114 | 5-Bromo-2-(4-iodo-2-methyl-phenylamino)-N-(3-piperidin-1-yl-propyl)-benzamide | 556* |
| 115 | N-{2-[Bis-(2-hydroxy-ethyl)-amino]-ethyl}-2-(4-iodo-2-methyl-phenylamino)-5-nitro-benzamide | 529* |
| 116 | 5-Chloro-2-(4-iodo-2-methyl-phenylamino)-N-(2-morpholin-4-yl-ethyl)-benzamide | 500* |
| 117 | 5-Chloro-N-(3-diethylamino-propyl)-2-(4-iodo-2-methyl-phenylamino)-benzamide | 500* |
| 118 | 5-Chloro-N-(2-diisopropylamino-ethyl)-2-(4-iodo-2-methyl-phenylamino)-benzamide | 514* |
| 119 | 5-Chloro-2-(4-iodo-2-methyl-phenylamino)-N-(3-piperidin-1-yl-propyl)-benzamide | 512* |
| 120 | 2-(4-Iodo-2-methyl-phenylamino)-5-nitro-N-(2-piperidin-1-yl-ethyl)-benzamide | 509* |
| 121 | 5-Bromo-2-(4-iodo-2-methyl-phenylamino)-N-(2-piperazin-1-yl-ethyl)-benzamide | 544* |
| 122 | N-(2-Diethylamino-ethyl)-5-fluoro-2-(4-iodo-2-methyl-phenylamino)-benzamide | 470* |
| 123 | 5-Bromo-N-(3-dimethylamino-propyl)-2-(4-iodo-2-methyl-phenylamino)-benzamide | 516* |
| 124 | N-(3-Hydroxy-propyl)-2-(4-iodo-2-methyl-phenylamino)-5-nitro-benzamide | 456* |
| 125 | 5-Fluoro-N-(3-hydroxy-propyl)-2-(4-iodo-2-methyl-phenylamino)-benzamide | 429* |
| 126 | N-(3-Diethylamino-propyl)-5-fluoro-2-(4-iodo-2-methyl-phenylamino)-benzamide | 484* |
| 127 | N-(3-Diethylamino-propyl)-2-(4-iodo-2-methyl-phenylamino)-5-nitro-benzamide | 511 * |
| 128 | 5-Bromo-2-(4-iodo-2-methyl-phenylamino)-N-(2-morpholin-4-yl-ethyl)-benzamide | 544* |
| 129 | 2-(4-Iodo-2-methyl-phenylamino)-5-nitro-N-(3-piperidin-1-yl-propyl)-benzamide | 523 * |
| 130 | 1-[5-Fluoro-2-(4-iodo-2-methyl-phenylamino)-phenyl]-(3-hydroxy-pyrrolidin-1-yl)-methanone | 439 |
| 131 | 5-Bromo-N-(2-diisopropylamino-ethyl)-2-(4-iodo-2-methyl-phenylamino)-benzamide | 558* |
| 132 | 5-Fluoro-2-(4-iodo-2-methyl-phenylamino)-N-(2-morpholin-4-yl-ethyl)-benzamide | 484* |
| 133 | 5-Fluoro-2-(4-iodo-2-methyl-phenylamino)-N-(3-piperidin-1-yl-propyl)-benzamide | 496* |
| 134 | 1-[5-Fluoro-2-(4-iodo-2-methyl-phenylamino)-phenyl]-[4-(2-hydroxy-ethyl)-piperazin-1-methanone | 482 |
| 135 | N-(3-Diethylamino-2-hydroxy-propyl)-5-fluoro-2-(4-iodo-2-methyl-phenylamino)-benzamide | 500* |
| 136 | [5-Chloro-2-(4-iodo-2-methyl-phenylamino)-benzoylamino]-acetic acid | 443 |
| 137 | 2-(4-Iodo-2-methyl-phenylamino)-5-nitro-N-(2-pyrrolidin-1-yl-ethyl)-benzamide | 495* |
| 138 | N-(3-Dimethylamino-propyl)-2-(4-iodo-2-methyl-phenylamino)-5-nitro-benzamide | 483* |
| 139 | N-(2-Diisopropylamino-ethyl)-5-fluoro-2-(4-iodo-2-methyl-phenylamino)-benzamide | 498* |
| 140 | 5-Fluoro-2-(4-iodo-2-methyl-phenylamino)-thiobenzoic acid S-phenethyl ester | 490 |
| 141 | 5-Chloro-2-(4-iodo-2-methyl-phenylamino)-thiobenzoic acid S-phenethyl ester | 506 |
| 142 | 5-Bromo-2-(4-iodo-2-methyl-phenylamino)-thiobenzoic acid S-benzyl ester | 536 |
| 143 | 2-(4-Iodo-2-methyl-phenylamino)-5-nitro-thiobenzoic acid S-benzyl ester | 503 |
| 144 | 5-Fluoro-2-(4-iodo-2-methyl-phenylamino)-thiobenzoic acid S-benzyl ester | 476 |
| 145 | 5-Chloro-2-(4-iodo-2-methyl-phenylamino)-thiobenzoic acid S-benzyl ester | 492 |
| 146 | N-Cyclopropyl-5-fluoro-2-(4-iodo-2-methyl-phenylamino)-benzamide | 409 |
| 147 | 5-Chloro-N-(2-hydroxy-ethyl)-2-(4-iodo-2-methyl-phenylamino)-benzamide | 429 |
| 148 | 5-Fluoro-N-(2-hydroxy-ethyl)-2-(4-iodo-2-methyl-phenylamino)-benzamide | 413 |
| 149 | N-Benzyloxy-5-fluoro-2-(4-iodo-2-methyl-phenylamino)-benzamide | 475 |
| 150 | N-Benzyloxy-5-bromo-2-(4-iodo-2-methyl-phenylamino)-benzamide | 593* |
| 151 | 2-(4-Iodo-2-methyl-phenylamino)-5-nitro-N-(4-sulfamoyl-benzyl)-benzamide | 567 |
| 152 | 5-Bromo-N-(2-hydroxy-ethyl)-2-(4-iodo-2-methyl-phenylamino)-benzamide | 473 |
| 153 | N-(2-Hydroxy-ethyl)-5-iodo-2-(4-iodo-2-methyl-phenylamino)-benzamide | 521 |
| 154 | N-(2-Hydroxy-ethyl)-2-(4-iodo-2-methyl-phenylamino)-5-nitro-benzamide | 440 |
| 155 | 2-(4-Iodo-2-methyl-phenylamino)-N-methyl-5-nitro-N-phenyl-benzamide | 486 |
| 156 | 5-Chloro-N-cyclopropyl-2-(4-iodo-2-methyl-phenylamino)-benzamide | 425 |
| 157 | 5-Fluoro-2-(4-iodo-2-methyl-phenylamino)-N-methyl-N-phenyl-benzamide | 459 |
| 158 | N-Allyl-5-fluoro-2-(4-iodo-2-methyl-phenylamino)-benzamide | 409 |
| 159 | N-Benzyloxy-5-iodo-2-(4-iodo-2-methyl-phenylamino)-benzamide | 583 |
| 160 | 5-Fluoro-2-(4-iodo-2-methyl-phenylamino)-N-(4-sulfamoyl-benzyl)-benzamide | 538 |
| 161 | N-Allyl-5-chloro-2-(4-iodo-2-methyl-phenylamino)-benzamide | 425 |
| 162 | N-Cyclopropyl-2-(4-iodo-2-methyl-phenylamino)-5-nitro-benzamide | 436 |
| 163 | 5-Bromo-N-cyclopropyl-2-(4-iodo-2-methyl-phenylamino)-benzamide | 469 |
| 164 | 5-Chloro-2-(4-iodo-2-methyl-phenylamino)-N-methyl-N-phenyl-benzamide | 475 |
| 165 | 5-Iodo-2-(4-iodo-2-methyl-phenylamino)-N-(4-sulfamoyl-benzyl)-benzamide | 646 |
| 166 | 5-Bromo-2-(4-iodo-2-methyl-phenylamino)-N-(4-sulfamoyl-benryl)-benzamide | 598 |
| 167 | N-Allyl-2-(4-iodo-2-methyl-phenylamino)-5-nitro-benzamide | 436 |
| 168 | 2-(4-Iodo-2-methyl-phenylamino)-5-nitro-N-(4-sulfamoyl-benzyl)-benzamide | 565 |
| 169 | N-Allyl-5-bromo-2-(4-iodo-2-methyl-phenylamino)-benzamide | 469 |
| 170 | 5-Fluoro-2-(4-iodo-2-methyl-phenylamino)-N-(3-methyl-benzyl)-benzamide | 473 |
| 171 | N-Cyclopropyl-5-iodo-2-(4-iodo-2-methyl-phenylamino)-benzamide | 517 |
| 172 | 5-Bromo-2-(4-iodo-2-methyl-phenylamino)-N-methyl-N-phenyl-benzamide | 519 |
| 173 | N-Benzyloxy-2-(4-iodo-2-methyl-phenylamino)-5-nitro-benzamide | 502 |
| 174 | N-Cyclohexyl-5-iodo-2-(4-iodo-2-methyl-phenylamino)-benzamide | 559 |
| 175 | N-Allyl-5-iodo-2-(4-iodo-2-methyl-phenylamino)-benzamide | 517 |
| 176 | 5-Iodo-2-(4-iodo-2-methyl-phenylamino)-N-(3-methyl-benzyl)-benzamide | 581 |
| 177 | 2-(4-Iodo-2-methyl-phenylamino)-N-(3-methyl-benzyl)-5-nitro-benzamide | 500 |
| 178 | 5-Iodo-2-(4-iodo-2-methyl-phenylamino)-N-methyl-N-phenyl-benzamide | 567 |
| 179 | N-Cyclohexyl-5-fluoro-2-(4-iodo-2-methyl-phenylamino)-benzamide | 451 |
| 180 | 5-Chloro-N-cyclohexyl-2-(4-iodo-2-methyl-phenylamino)-benzamide | 467 |
| 181 | 5-Bromo-2-(4-iodo-2-methyl-phenylamino)-N-(3-methyl-benzyl)-benzamide | 533 |
| 182 | 5-Bromo-N-cyclohexyl-2-(4-iodo-2-methyl-phenylamino)-benzamide | 511 |
| 183 | 5-Chloro-2-(4-iodo-2-methyl-phenylamino)-N-(3-methyl-benzyl)-benzamide | 489 |
| 184 | N-Cyclohexyl-2-(4-iodo-2-methyl-phenylamino)-5-nitro-benzamide | 478 |
| 185 | N-Benzyloxy-5-bromo-2-(4-iodo-2-methyl-phenylamino)-benzamide | 538 |
| 186 | N-Benzyloxy-5-fluoro-2-(4-iodo-2-methyl-phenylamino)-benzamide | 477 |
| 187 | 5-Chloro-N-(2-hydroxy-ethyl)-2-(4-iodo-2-methyl-phenylamino)-benzamide | 431 |
| 188 | 5-Bromo-N-(2-hydroxy-ethyl)-2-(4-iodo-2-methyl-phenylamino)-benzamide | 475 |
| 189 | 2-(4-Iodo-2-methyl-phenylamino)-N-methyl-5-nitro-N-phenyl-benzamide | 488 |
| 190 | 5-Chloro-2-(4-iodo-2-methyl-phenylamino)-N-methyl-N-phenyl-benzamide | 477 |
| 191 | N-(2-Hydroxy-ethyl)-5-iodo-2-(4-iodo-2-methyl-phenylamino)-benzamide | 523 |
| 192 | 5-Chloro-N-cyclopropyl-2-(4-iodo-2-methyl-phenylamino)-benzamide | 425 |
| 193 | N-Allyl-5-chloro-2-(4-iodo-2-methyl-phenylamino)-benzamide | 427 |
| 194 | 5-Fluoro-2-(4-iodo-2-methyl-phenylamino)-N-methyl-N-phenyl-benzamide | 461 |
| 195 | N-(2-Hydroxy-ethyl)-2-(4-iodo-2-methyl-phenylamino)-5-nitro-benzamide | 442 |
| 196 | 5-Fluoro-N-(2-hydroxy-ethyl)-2-(4-iodo-2-methyl-phenylamino)-benzamide | 415 |
| 197 | 5-Bromo-N-cyclopropyl-2-(4-iodo-2-methyl-phenylamino)-benzamide | 472 |
| 198 | N-Cyclopropyl-5-fluoro-2-(4-iodo-2-methyl-phenylamino)-benzamide | 411 |
| 199 | 5-Fluoro-2-(4-iodo-2-methyl-phenylamino)-N-(4-sulfamoyl-benzyl)-benzamide | 540 |
| 200 | N-Cyclopropyl-2-(4-iodo-2-methyl-phenylamino)-5-nitro-benzamide | 438 |
| 201 | N-Allyl-5-fluoro-2-(4-iodo-2-methyl-phenylamino)-benzamide | 411 |
| 202 | N-Benzyloxy-5-iodo-2-(4-iodo-2-methyl-phenylamino)-benzamide | 585 |
| 203 | N-Allyl-5-bromo-2-(4-iodo-2-melhyl-phenylamino)-benzamide | 472 |
| 204 | 5-Bromo-2-(4-iodo-2-methyl-phenylamino)-N-(4-sulfamoyl-benzyl)-benzamide | 601 |
| 205 | 5-Bromo-2-(4-iodo-2-methyl-phenylamino)-N-methyl-N-phenyl-benzamide | 522 |
| 206 | N-Allyl-2-(4-iodo-2-methyl-phenylamino)-5-nitro-benzamide | 438 |

| | | |
|---|---|---|
| * M+H | | |

### EXAMPLE 207

### Preparation of [4-Chloro-2-(1H-tetrazol-5-yl)phenyl]-(4-iodo-2-methyl-phenyl)amine

### Step a: Preparation of 5-Chloro-2-fluoro-benzaldehyde

To a solution of 1-chloro-4-fluorobenzne (13.06 g, 0.1 mol) in THF (180 mL), at -78°C, LDA (2 M solution in THF, 50 mL, 0.1 mol) was added dropwise. After stirring at -78°C for 1.5 hours, DMF (8 mL) was added to the reaction mixture and allowed to warm up to room temperature overnight. The reaction mixture was partitioned between water and Et₂O. The Et₂O layer was dried (MgSO₄) and the solvent removed in vacuum to give 14.95 g (94%) yield of crude aldehyde:
¹H NMR (CDCl₃): δ, 10.3 (s, -C(=O)H).

### Step b: Preparation of 5-Chloro-2-fluoro-benzaldehyde oxime

A solution of 5-chloro-2-fluoro-benzaldehyde (10 g, 0.0631 mol), hydroxylamine hydrochloride (6.57 g, 0.0946 mol) and pyridine (8.3 mL, 0.1010 mol) in EtOH (100 mL) was heated at 75°C (oil bath temperature) for 1 hour and the solvent removed under vacuum to give an oil. The oil was partitioned between water and CH₂Cl₂. The CH₂Cl₂ layer was dried (MgSO₄) and the solvent removed under vacuum to give crude aldoxime as a solid. The solid was purified by medium pressure liquid chromatography on silica. Elution with CH₂Cl₂ gave 4.87g (28%) of the aldoxime as white solid: mp 95-97°C;

| Analysis calculated for C₇H₅NOFCl: | | | |
|---|---|---|---|
| | C, 48.44; | H, 2.90; | N, 8.07. |
| Found: | C, 48.55; | H, 2.69, | N, 7.90. |

### Step c: Preparation of 5-Chloro-2-fluoro-benzonirile

A solution of the 5-chloro-2-fluoro-benzaldehyde oxime (3.15 g, 0.0182 mol) in acetic anhydride (150 mL) was refluxed for 16 hours. The reaction mixture was cooled to room temperature and poured into saturated aqueous NaHCO₃ (200 mL) solution. The mixture was extracted with Et₂O. The Et₂O layer was dried (K₂CO₃) and the solvent removed to give the product as an oily solid. The product was used without further purification in the next step.

### Step d: Preparation of 5-(5-Chloro-2-fluoro-phenyl)-1H-tetrazole

A mixture of 5-chloro-2-fluoro-benzonitrile (2.84 g, 0.01823 mol), butanol (15 mL), sodium azide (1.543 g, 0.0237 mol), acetic acid (1.36 mL, 0.0237 mol) was refluxed for 24 hours. The reaction mixture was cooled to room temperature, additional 1.543 g sodium azide added, and the reaction mixture refluxed for additional 24 hours. After cooling to room temperature, Et₂O (100 mL) and 10% aqueous NaOH (200 mL) were added sequentially. The mixture was vigorously stirred. The aqueous layer was separated, cooled with ice-methanol bath (-15°C) and acidified to pH 1 with conc. HCl. A gray solid precipitated. The solid was dried in vacuum at 50°C to give 1.76 g (49%) of 5-(5-chloro-2-fluoro-phenyl)-1H-tetrazole: mp partial melt at 110°C, complete melting at 124°C);
¹H (400 Mz, CDCl₃): δ 8.19-8.08 (m, 1H), 7.77-7.71 (m, 1H), 7.61-7.52 (m, 1H);
¹³C (100 Mz, CDCl₃): δ 159.00, 156.49, 140.88, 133..02, 132.93, 130.73, 129.23, 129.21, 129.08, 126.05, 118.96, 118.73, 114.50;
MS (CI) M+1 = 199 (100), M = 198 (6).

### Step e: Preparation of [4-Chloro-2-(1H-tetrazol-5-yl)phenyl]-(4-iodo-2-methyl-phenyl)amine

To a solution of 2-methyl-4-iodoaniline (3.52 g, 0.0151 mol) in THF (25 mL) at -78°C, LDA (2 molar solution in THF, 11.33 mL, 0.02267 mol) was added dropwise. After stirring for 0.5 hours; a solution of 1-(tetrazol-5-yl)-2-fluoro-5-chlorobenzene (1.5 g, 0.00756 mol) in THF (15 mL) was added dropwise. The reaction was stirred for 16 hours as it warmed up to room temperature. The reaction mixture was quenched with aqueous conc. NH₄Cl solution and extracted with CH₂Cl₂. The organic layer was dried (MgSO₄) and the solvent removed giving a crude product as an oil. The oil with CH₂Cl₂->CH₂Cl₂:MeOH (9.7:0.3) gave 1.5 g (48%) of the desired product: mp 205-208;
¹H (400 Mz, DMSO): δ 9.13 (s, 1H), 8.00-7.99 (s, 1H), 7.69 (s, 1H), 7.55-7.52 (m, 1H), 7.43-7.40 (m, 1H), 7.12-7.05 (m, 1H), 2.24 (s, 3H);
¹³C (100 Mz, CDCl₃): δ 141.87, 139.28, 138.88, 135.47, 133.71, 131.65, 128.15, 123.69, 121.94, 116.68, 87.79, 17.22;
MS (CI) M+2 = 413 (44), M+1 = 412 (85), M = 411 (100).

| Analysis calculated for C₁₄H₁₁N₅Cll·0.5H₂O: | | | |
|---|---|---|---|
| | C, 39.97; | H, 2.87; | N, 16.65. |
| Found: | C, 38.87, | H, 2.77; | N, 16.47. |

The following tetrazole substituted phenylamines were prepared by following the general procedure of Example 207.

### EXAMPLE 208

### (4-Iodo-2-methyl-phenyl)-[2-(1H-tetrazol-5-yl)-phenyl]amine, mp 231°C (dec)

### EXAMPLE 209

### [4-Nitro-2-(1H-tetrazol-5-yl) phenyl]-(4-iodo-2-methyl-phenyl)-amine, mp 205-208°C.

The invention compounds are useful in treating cancer and other proliferative diseases by virtue of their selective inhibition of the dual specificity protein kinases MEK₁ and MEK₂. The invention compound has been evaluated in a number of biological assays which are normally utilized to establish inhibition of proteins and kinases, and to measure mitogenic and metabolic responses to such inhibition.

### EXAMPLES 210-224

Additional invention compounds which were prepared by the general methods described above are:

| Example No. | Compound | MP °C |
|---|---|---|
| 210 | 2-(2-Chloro-4-iodo-phenylamino)-3-fluoro-4-(2-morpholin-4-yl-ethylamino)-5-nitro-benzoic acid | 239-241 DEC |
| 211 | 4-Amino-2-(2-chloro-4-iodo-phenylamino)-3-fluoro-5-nitro-benzoic acid | >270 |
| 212 | 2,4-Bis-(2-chloro-4-iodo-phenylamino)-3-fluoro-5-nitro-benzoic acid | >265 DEC |
| 213 | 4-Fluoro-2-(4-iodo-2-methyl-phenylamino)-5-nitro-benzoic acid | 218-225 DEC |
| 214 | 2-(2,6-Difluoro-4-iodo-phenylamino)-3,4-difluoro-benzoic acid | 247-249 |
| 215 | 2-(2-Chloro-4-iodo-phenylamino)-4-nitro-benzoic acid | 267-269 |
| 216 | 2-(2,4-Diiodo-phenylamino)-4-fluoro-benzoic acid | 260-261 |
| 217 | 2-(2-Bromo-4-iodo-phenylamino)-4-fluoro-benzoic acid | 259-262 |
| 218 | 4-Fluoro-2-(2-fluoro-4-iodo-phenylamino)-benzoic acid | 215-217 |
| 219 | 2-(2-Chloro-4-iodo-phenylamino)-4-fluoro-benzoic acid | 242-247 |
| 220 | S-Bromo-2-(2-chloro-4-iodo-phenylamino)-3,4-difluoro-benzoic acid | 312.5-318 |
| 221 | 2,3,5-Trifluoro-6-(4-iodo-2-methyl-phenylamino)-4-(4-methyl-piperazin-1-yl)-benzoic acid methyl ester dihydrofluoride salt | 118-121 |
| 222 | 5-Bromo-3,4-difluoro-2-(4-iodo-2-methyl-phenylamino)-N-(4-methyl-piperazin-1-yl)-benazmide | 214-217 DEC |
| 223 | 5-Bromo-3,4-difluoro-2-(4-iodo-2-methyl-phenylamino)-benzoic acid N',N'-dimethyl-hydrazide | 154-175 DEC |
| 224 | 4-Fluoro-2-(4-iodo-2-methyl-phenylamino)-benzoic acid hydrazide | 153.5-156 |

### Enzyme Assays

### Cascade assay for inhibitors of the MAP kinase pathway

Incorporation of ³²P into myelin basic protein (MBP) was assayed in the presence of a glutathione S-transferase fusion protein containing p44MAP kinase (GST-MAPK) and a glutathione S-transferase fusion protein containing p45MEK (GST-MEK). The assay solution contained 20 mM HEPES, pH 7.4, 10 mM MgCl₂, 1-mM MnCl₂, 1 mM EGTA, 50 µM [γ-³²P]ATP, 10 µg GST-MEK, 0.5 µg GST-MAPK and 40 µg MBP in a final volume of 100 µL. Reactions were stopped after 20 minutes by addition of trichloroacetic acid and filtered through a GF/C filter mat. ³²P retained on the filter mat was determined using a 1205 Betaplate. Compounds were assessed at 10 µM for ability to inhibit incorporation of ³²P.

To ascertain whether compounds were inhibiting GST-MEK or GST MAPK, two additional protocols were employed. In the first protocol, compounds were added to tubes containing GST-MEK, followed by addition of GST-MAPK, MBP and [γ-³²P]ATP. In the second protocol, compounds were added to tubes containing both GST-MEK and GST-MAPK, followed by MBP and [γ-³²P]ATP, Compounds that showed activity in both protocols were scored as MAPK inhibitors, while compounds showing activity in only the first protocol were scored as MEK inhibitors.

### In vitro MAP kinase assay

Inhibitory activity was also confirmed in direct assays. For MAP kinase, 1 µg GST-MAPK was incubated with 40 µg MBP for 15 minutes at 30°C in a final volume of 50 µL containing 50 mM Tris (pH 7.5), 10 µM MgCl₂, 2 µM EGTA, and 10 µM [γ-³²P]ATP. The reaction was stopped by addition of Laemmli SDS sample buffer and phosphorylated MBP resolved by electrophoresis on a 10% polyacrylamide gel. Radioactivity incorporated into MBP was determined by autoradiography, and subsequently by excision of the bands followed by scintillation counting.

### In vitro MEK assay

For evaluation of direct MEK activity, 10 µg GST-MEK₁ was incubated with 5 µg of a glutathione S-transferase fusion protein containing p44MAP kinase with a lysine to alanine mutation at position 71 (GST-MAPK-KA). This mutation eliminates kinase activity of MAPK, so only kinase activity attributed to the added MEK remains. Incubations were 15 minutes at 30°C in a final volume of 50 µL containing 50 mM Tris (pH 7.5), 10µM MgCl₂, 2µM EGTA, and 10 µM [γ-³²P]ATP. The reaction was stopped by addition of Laemmli SDS sample buffer and phosphorylated GST-MAPK-KA was resolved by electrophoresis on a 10% polyacrylamide gel. Radioactivity incorporated into GST-MAPK-KA was determined by autoradiography, and subsequently by excision of the bands followed by scintillation counting. Additionally, an artificially activated MEK was utilized that contained serine to glutamate mutations at positions 218 and 222 (GST-MEK-2E). When these sites are phosphorylated, MEK activity is increased. Phosphorylation of these sites can be mimicked by mutation of the serine residues to glutamate. For this assay, 5 µg GST-MEK-2E was incubated with 5 µg GST-MAPK-KA for 15 minutes at 30°C in the same reaction buffer as described above. Reactions were terminated and analyzed as above.

### Whole cell MAP kinase assay

To determine if compounds were able to block activation of MAP kinase in whole cells, the following protocol was used: Cells were plated in multi-well plates and grown to confluence. Cells were then serum-deprived overnight. Cells were exposed to the desired concentrations of compound or vehicle (DMSO) for 30 minutes, followed by addition of a growth factor, for example, PDGF (100 ng/mL). After a 5-minute treatment with the growth factor, cells were washed with PBS, then lysed in a buffer consisting of 70 mM NaCl, 10 mM HEPES (pH 7.4), 50 mM glycerol phosphate, and 1% Triton X-100. Lysates were clarified by centrifugation at 13,000 × g for 10 minutes. Five micrograms of the resulting supernatants were incubated with 10 µg microtubule associated protein-2 (Map2) for 15 minutes at 30°C in a final volume of 25 µL containing 50 mM Tris (pH 7.4), 10 mM MgCl₂, 2 mM EGTA and 30 µM [γ-³²P]ATP, Reactions were terminated by addition of Laemmli sample buffer. Phosphorylated Map2 was resolved on 7.5% acrylamide gels and incorporated radioactivity determined by autoradiography and subsequent excision of the bands followed by scintillation counting.

### Immunoprecipitation and antiphosphotyrosine immunoblots

To determine the state of tyrosine phosphorylation of cellular MAP kinase, cells were lysed, endogenous MAP kinase was immunoprecipitated with a specific antibody, and the resulting immunoprecipitate analyzed for the presence of phosphotyrosine as follows: confluent cells were serum-deprived overnight and treated with compounds and growth factors as described above. Cells were then scraped and pelleted at 13,000 × g for 2 minutes. The resulting cell pellet was resuspended and dissolved in 100 µL of 1% SDS containing 1 mM NaVO₄. Following alternate boiling and vortexing to denature cellular protein, 900 µL RIPA buffer (50 mM Tris (pH 7.4), 150 mM NaCl, 1% Triton X-100, 0.1% deoxycholate, and 10 mM EDTA) was added. To this mixture was added 60 µL agarose beads coupled with rabbit immunoglobulin G and 60 µL Pansorbin cells in order to clear the lysate of nonspecific binding proteins. This mixture was incubated at 4°C for 15 minutes then centrifuged at 13,000 × g for 10 minutes. The resulting supernatant was transferred to fresh tubes and incubated with 10 µL of a polyclonal antisera raised against a fragment of MAP kinase for a minimum of 1 hour at 4°C. Seventy microliters of a slurry of agarose beads coupled with protein G and protein A was added and the incubation continued for an additional 30 minutes at 4°C. The beads were pelleted by centrifugation at 13,000 × g for 5 minutes and washed three times with 1 mL RIPA buffer. Laemmli sample buffer was added to the final bead pellet. This mixture was boiled for 5 minutes then resolved on a 10% acrylamide gel. Proteins on the gel were transferred to a nitrocellulose membrane and nonspecific binding sites on the membrane blocked by incubation with 1% ovalbumin and 1% bovine serum albumin in TBST (150 mM NaCl, 10 mM Tris (pH 7.4), and 0.05% Tween 20). The membrane was then incubated with a commercially available antibody directed against phosphotyrosine. Antibody bound on the membrane was detected by incubation with ¹²⁵I-protein A, followed by autoradiography.

### Cell Growth Assays

### ³H-Thymidine incorporation

Cells were plated in multi-well plates and grown to near confluence. The media was then removed and replaced with growth media containing 1% bovine serum albumin. After 24-hour serum starvation, compounds and specific growth factors were added and incubations continued for an additional 24 hours. During the final 2 hours, ³H-thymidine was added to the medium. To terminate the incubations, the medium was removed and cell layers washed twice with ice-cold phosphate-buffered saline. After the final wash, ice-cold 5% trichloroacetic acid was added and the cells incubated for 15 minutes at room temperature. The trichloroacetic acid solution was then removed and the cell layer washed three times with distilled water. After the final wash, the cell layer was solubilized by addition of 2% sodium dodecylsulfate. Radioactivity in this solution was determined by scintillation counting.

In 3T3-L1 adipocyte cells, in which the inhibition blocks MAPK activation by insulin with an IC₅₀ of 3 µM, the compound had no effect on the insulin stimulated uptake of radiolabeled 2-deoxyglucose, or on the insulin-stimulated synthesis of either lipid or glycogen at 10 µM concentration. This demonstrates that the inhibitor shows selectivity between the mitogenic and metabolic effects of insulin, and demonstrates that the inhibitor will show less toxicity than an inhibitor which does not show this surprising selectivity.

### Monolayer growth

Cells were plated into multi-well plates at 10 to 20,000 cells/mL. Forty-eight hours after seeding, compounds were added to the cell growth medium and incubation was continued for 2 additional days. Cells were then removed from the wells by incubation with trypsin and enumerated with a Coulter counter.

### Growth in soft-agar

Cells were seeded into 35-mm dishes at 5 to 10,000 cells/dish using growth medium containing 0.3% agar. After chilling to solidify the agar, cells were transferred to a 37°C incubator. After 7 to 10 days growth, visible colonies were manually enumerated with the aid of a dissecting microscope.
Order of addition experiments established that the invention compounds are inhibiting MEK and not MAP kinase. Experiments looking at the phosphorylation of a kinase defective mutant of MAP kinase as substrate (so that there can be no autophosphorylation of the MAP kinase to complicate interpretation) confirms that the inhibitor inhibits MEK with an IC₅₀ essentially identical to that produced in the cascade assay.

Kinetic analysis demonstrates that the invention compounds are not competitive with ATP. Thus, they do not bind at the ATP binding site of the enzyme, which is probably the explanation as to why these compounds do not show the nonspecific kinase inhibitory activity typical of most kinase inhibitors, which do bind at the ATP binding site and which are ATP competitive. The in vitro and in vivo biological activity of several representative compounds of Formula I in the foregoing assays is presented in Table 1.

**Table 1**

| Compound of Example No. | In Vitro | | In Vivo (Cell Culture) | |
|---|---|---|---|---|
| | % Inhibition | IC₅₀ µM | % Inhibition | IC₅₀ µM |
| 1 | | 0.019 | | |
| 2 | | 0.014 | | 3 |
| 3 | | 0.0111 | | 10 |
| 4 | | 0.005 | | 1 |
| 5 | | 0.066 | | |
| 6 | | 0.071 | | |
| 7 | | 0.072 | | |
| 8 | | 0.086 | | |
| 9 | | 0.097 | | |
| 10 | | 0.101 | | |
| 11 | | 0.128 | | |
| 12 | | 0.135 | | |
| 13 | | 0.178 | | |
| 14 | | 0.179 | | |
| 15 | | 0.194 | | |
| 16 | | 0.323 | | |
| 17 | | 0.434 | | |
| 18 | | 0.446 | | |
| 19 | | 0.524 | 50% at 30 µM | |
| 20 | | 0.557 | | |
| 21 | | 0.569 | | |
| 22 | | 1.581 | 30% at 30 µM | |
| 23 | | 1.588 | | |
| 24 | | 1.944 | | |
| 25 | | 2.363 | | |
| 26 | | 2.609 | 50% at 30 µM | |
| 27 | | 2.269 | | |
| 28 | | 3.670 | | |
| 29 | | 5.331 | | |
| 30 | 105 | | | 10 |
| 31 | | 0.226 | | |
| 32 | | 0.028 | | |
| 33 | | 0.052 | | |
| 34 | | 0.098 | | |
| 35 | | 0.121 | | |
| 36 | | 0.129 | | |
| 37 | | 0.237 | | |
| 38 | | 0.412 | | |
| 39 | | 0.497 | | |
| 40 | | 0.651 | 30% at 30 µM | |
| 41 | | 0.872 | | |
| 42 | | 0.920 | | |
| 43 | | >1.000 | | |
| 44 | | 1.481 | | |
| 45 | | 1.755 | | |
| 46 | | 1.814 | | |
| 47 | | 1.911 | | |
| 48 | | 1.945 | | |
| 49 | | 0.418 | | 3 |
| 50 | | 0.179 | | |
| 51 | | 0.887 | | |
| 52 | | 2.346 | | |
| 53 | | 0.047 | | 0.54 |
| 54 | | 0.158 | | |
| 55 | | 0.114 | | |
| 57 | | 0.399 | | |
| 89 | | 0.186 | | |
| 89 | | 0.614 | | |
| 90 | | 0.604 | | |
| 91 | | 2.071 | | |
| 92 | | 0.253 | | |
| 93 | | 0.521 | | |
| 95 | | 1.001 | | |
| 96 | | 0.374 | | |
| 100 | | 1.994 | | |
| 184 | | 0.278 | | |
| 186 | | 0.555 | | |
| 187 | | 0.561 | | |
| 188 | | 0.771 | | |
| 189 | | 0.859 | | |
| 190 | | 0.921 | | |
| 191 | | 1.355 | | |
| 192 | | 1.797 | | |
| 193 | | 2.902 | | |
| 194 | | 4.952 | | |
| 195 | | 12.831 | | |
| 208 | | 1.215 | | |
| 209 | | 1.372 | | |
| 211 | | >0.1 | | |
| 212 | | 0.034 | | |
| 213 | | 0.062 | | |
| 214 | | 0.303 | | |
| 215 | | 0.031 | | |
| 216 | | 1.000 | | |
| 217 | | >1.00 | | |
| 218 | | 0.051 | | |
| 219 | | 0.108 | | |
| 220 | | 0.029 | | |
| 221 | | 0.002 | | |
| 222 | | 0.085 | | |
| 223 | | 0.043 | | |
| 224 | | 0.028 | | |

The invention compounds will be utilized for preparing a pharmaceutical composition for treating subjects suffering from cancer and other proliferative diseases, immunodeficiency, and certain degenerative diseases, and in need of treatment. The compounds are ideally suited for preparing a pharmaceutical composition for treating psoriasis, restenosis, autoimmune disease, and atherosclerosis. The compounds will generally be utilized as a pharmaceutical formulation, in which the compound of Formula I is present in a concentration of about 5% to about 95% by weight. The compounds can be formulated for convenient oral, parenteral, topical, rectal, or like routes of administration. The compound will be formulated with common diluents, excipients, and carriers routinely utilized in medicine, for instance, with polyols such as glycerin, ethylene glycol, sorbitol 70; mono- and difatty acid esters of ethylene glycol. Starches and sugars such as corn starch, sucrose, lactose, and the like, can be utilized for solid preparations. Such solid formulations can be in the form of tablets, troches, pills, capsules, and the like. Flavoring agents such as peppermint, oil of wintergreen, and the like can be incorporated.

Typical doses of active compound are those that are effective to treat the cancer or other proliferative disorder afflicting the mammal. Doses will generally be from about 0.1 mg per kilogram body weight to about 500 mg per kilogram body weight. Such doses will be administered from one to about four times a day, or as needed to effectively treat the cancer, psoriasis, restenosis, or other proliferative disorder.

A preferred method for delivering the invention compound is orally via a tablet, capsule, solution, or syrup. Another method is parenterally, especially via intravenous infusion of a solution of the benzopyran in isotonic saline or 5% aqueous glucose.

Following are typical formulations provided by the invention.

### EXAMPLE 225

**Preparation of 50-mg Tablets**

| Per Tablet | | Per 10,000 Tablets |
|---|---|---|
| 0.050 g | 4-fluoro-2-(4-iodo-2-methyl-phenylamino)-benzoic acid | 500 g |
| 0.080 g | lactose | 800 g |
| 0.010 g | corn starch (for mix) | 100 g |
| 0.008 g | corn starch (for paste) | 80 g |
| 0.002 g | magnesium stearate (1%) | 20 g |
| 0.150 g | | 1500 g |

The benzoic acid, lactose, and corn starch (for mix) are blended to uniformity- The corn starch (for paste) is suspended in 600 mL of water and heated with stirring to form a paste. The paste is used to granulate the mixed powders. The granules are passed through a #8 screen and dried at 120°F. The dry granules are passed through a #16 screen. The mixture is lubricated with 1% magnesium stearate and compressed into tablets. The tablets are administered to a mammal for inhibiting MEK enzymes and treating restenosis, atherosclerosis, and psoriasis.

### EXAMPLE 226

**Preparation of Oral Suspension**

| Ingredient | Amount |
|---|---|
| 5-Chloro-2-(4-iodo-2-methyl-phenylamino)-N-(methyl)-benzamide | 500 mg |
| Sorbitol solution (70% NF) | 40 mL |
| Sodium benzoate | 150 mg |
| Saccharin | 10 mg |
| Red dye | 10 mg |
| Cherry flavor | 50 mg |
| Distilled water qs ad | 100 mL |

The sorbitol solution is added to 40 mL of distilled water and the benzamide derivative is suspended therein. The saccharin, sodium benzoate, flavor, and dye are added and dissolved. The volume is adjusted to 100 mL with distilled water. Each milliliter of syrup contains 5 mg of the invention compound. The syrup is administered to a mammal for treating proliferative disease, especially breast cancer and skin cancer.

### EXAMPLE 227

### Preparation of Parenteral Solution

In a solution of 700 mL of propylene glycol and 200 mL of water for injection is added 20.0 g of 4-fluoro-2-(4-bromo-2-methyl-phenylamino)-benzyl alcohol. The volume of the solution is adjusted to 1000 mL by addition of water for injection. The formulation is heat sterilized, filled into 50-mL ampoules each containing 2.0 mL (40 mg of 4-fluoro-2-(4-bromo-2-methyl-phenyl amino)-benzyl), and sealed under nitrogen.

The invention compound thus formulated will be administered to a mammal in need of treatment for a proliferative disorder such as cancer, psoriasis, restenosis, atherosclerosis, autoimmune disease, and other immunodeficient diseases and degenerative disorders, at a rate and dose effective to treat the condition. An "antiproliferative amount" of an invention compound is that quantity of compound that inhibits or reduces the rate of proliferation of target cells. Typical cancers to be treated according to this invention include breast cancer, colon cancer, prostate cancer, skin cancer, and the like. The invention compound is especially well-suited for use in combination with radiation for treating cancer. The compound is well-suited to the treatment of psoriasis, restenosis, and atherosclerosis, and to inhibiting the activity of MEK enzymes, especially MEK₁ and MEK₂. All that is required to inhibit these enzymes is to administer to a mammal an MEK inhibiting amount of a compound of the invention. An "MEK inhibiting amount" of an invention compound is an amount that when administered to a mammal causes a measurable inhibition of the MEK enzyme. Typical MEK inhibiting amounts will be from about 0.1 µg to about 500 mg of active compound per kilogram body weight. For treating the proliferative diseases mentioned above, typical doses will be from about 0.1 to about 50 mg/kg, normally given from one to about four times per day.

## Claims

1. A compound of Formula I 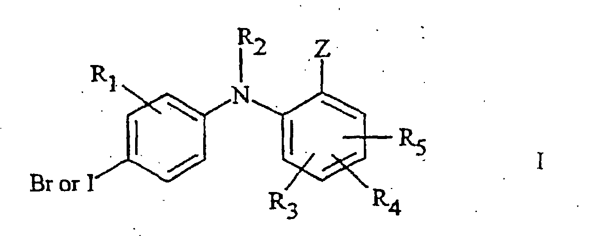 wherein:
R₁ is hydroxy, C₁-C₈ alkyl, C₁-C₈ alkoxy, halo, trifluoromethyl, or CN;
R₂ is hydrogen;
R₃, R₄, and R₅ independently are hydrogen, hydroxy, halo, trifluoromethyl, C₁-C₈ alkyl, C₁-C₈ alkoxy, nitro, CN, or -(O or NH)ₘ -(CH₂)ₙ-R₉, where R₉ is hydrogen, hydroxy, COOH, or NR₁₀R₁₁;
n is 0-4;
m is 0 or 1;
R₁₀ and R₁₁ independently are hydrogen or C₁-C₈ alkyl, or taken together with the nitrogen to which they are attached can complete a 3-10 member cyclic ring optionally containing 1, 2, or 3 additional heteroatoms selected from O, S, NH, or N-C₁-C₈ alkyl;
Z is COOR₇, tetrazolyl, CONR₆R₇, CONHNR₁₀R₁₁, or CH₂OR₇;
R₆ and R₇ independently are hydrogen, C₁-C₈ alkyl, C₂-C₈ alkenyl, C₂-C₈ alkynyl, 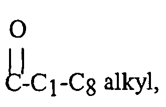 aryl, heteroaryl, or C₃-C₁₀ cycloalkyl optionally containing one, two, or three heteroatoms selected from O, S, NH, or N alkyl; or R₆ and R₇ together with the nitrogen to which they are attached complete a 3-10 member cyclic ring optionally containing 1, 2, or 3 additional heteroatoms selected from O, S, NH, or N alkyl; and wherein any of the foregoing alkyl, alkenyl, and alkynyl groups can be unsubstituted or substituted by halo, hydroxy, alkoxy, amino, alkylamino, dialkylamino, cycloalkyl, aryl, aryloxy, heteroaryl, or heteroaryloxy, and the pharmaceutically acceptable salts thereof, with the proviso that compounds of formula I are not 2'-4-4'-tribromodiphenylamine-2-carboxylic acid, methyl ester of 2'-4-4'-iribromodiphenylamine-2-carboxylic acid or 2[(2,4-dibromophenyl)amino]-5-bromo-benzoic acid.

2. A compound according to Claim 1 wherein R₁ is CH₃ or halo.

3. A compound according to Claim 2 wherein Z is COOR₇, tetrazolyl, or a salt thereof.

4. A compound according to Claim 3 which is
[4-Chloro-2-(1H-tetrazol-5-yl)-phenyl]-(4-iodo-2-methyl-phenyl)-amine;
(4-Iodo-2-methyl-phenyl)-[2-(1H-tetrazol-5-yl)-phenyl]amine; and
[4-Nitro-2-(1H-tetrazol-5-yl)-phenyl]-(4-iodo-2-methyl-phenyl)-amine.

5. A compound according to Claim 3 having the formula

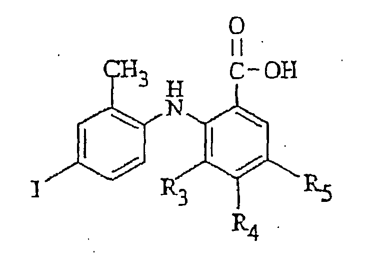

6. A compound of Claim 5 wherein R₃ is hydrogen, fluoro, or chloro; R₄ is hydrogen, fluoro, chloro, or nitro; and R₅ is hydrogen, chloro, fluoro, bromo, nitro, or methoxy.

7. A compound of Claim 6 which is
4-Fluoro-2-(4-iodo-2-methylphenylamino)benzoic acid;
3,4,5-Trifluoro-2-(4-iodo-2-methyl-phenylamino)-benzoic acid;
3,4-Difluoro-2-(4-iodo-2-methyl-phenylamino)-benzoic acid;
5-Bromo-3,4-difluoro-2-(4-iodo-2-methyl-phenylamino)-benzoic acid;
5-Chloro-2-(4-iodo-2-methyl-phenylamino)-benzoic acid;
Sodium 5-Chloro-2-(4-iodo-2-methyl-phenylamino)-benzoate;
5-Bromo-2-(4-iodo-2-methyl-phenylamino)-benzoic acid;
2-(4-Iodo-2-methyl-phenylamino)-5-nitro-benzoic acid;
4-Chloro-2-(4-iodo-2-methyl-phenylamino)-benzoic acid;
2-(4-Iodo-2-methyl-phenylamino)-benzoic acid;
5-Fluoro-2-(4-iodo-2-methyl-phenylamino)-benzoic acid;
2-(4-Iodo-2-methyl-phenylamino)-4-nitro-benzoic acid; and
4-Fluoro-2-(4-iodo-2-methyl-phenylamino)-5-nitro-benzoic acid.

8. A compound of Claim 3 having the formula

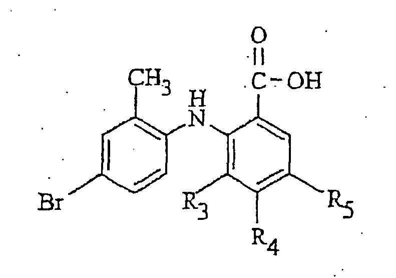

9. A compound of Claim 8 wherein R₃ is hydrogen, chloro, or fluoro; R₄ is hydrogen, chloro, fluoro, or nitro; R₅ is hydrogen, chloro, fluoro, bromo, nitro, or methoxy.

10. A compound of Claim 1 which is
2-(4-Bromo-2-methyl-phenylamino)-4-fluoro-benzoic acid;
2-(2-Bromo-4-iodo-phenylamino)-5-nitro-benzoic acid;
2-(4-Bromo-2-methyl-phenylamino)-3,4-difluoro-benzoic acid;
2-(2-Chloro-4-iodo-phenylamino)-3-fluoro-4-(2-morpholin-4-yl-ethylamino)-5-nitro-benzoic acid;
4-Amino-2-(2-chloro-4-iodo-phenylamino)-3-fluoro-5-nitro-benzoic acid;
2-(2-Chloro-4-iodo-phenylamino)-4-nitro-benzoic acid;
2-(2,4-Diiodo-phenylamino)-4-fluoro-benzoic acid;
2-(2-Bromo-4-iodo-phenylamino)-4-fluoro-benzoic acid;
4-Fluoro-2-(2-fluoro-4-iodo-phenylamino)-benzoic acid;
2-(2-Chloro-4-iodo-phenylamino)-4-fluoro-benzoic acid;
5-Bromo-2-(2-chloro-4-iodo-phenylamino)-3,4-difluoro-benzoic acid;
5-Iodo-2-(4-iodo-2-methyl-phenylamino)-benzoic acid;
2-(4-Iodo-phenylamino)-5-methoxy-benzoic acid;
5-Methyl-2-(4-iodo-2-methyl-phenylamino)-benzoic acid;
5-Bromo-3,4-difluoro-2-(4-iodo-2-methyl-phenylamino)-benzoic acid N',N'-dimethyl-hydrazide; and
4-Fluoro-2-(4-iodo-2-methyl-phenylamino)-benzoic acid hydrazide.

11. A compound of Claim 2 wherein Z is CONR₆R₇

12. A compound of Claim 11 having the formula 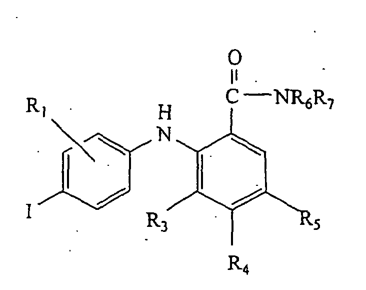 wherein:
R₁ is CH₃ or halo.

13. A compound of Claim 12 wherein R₃ is hydrogen, chloro, or fluoro; R₄ is hydrogen, chloro, fluoro, or nitro; and R₅ is hydrogen, chloro, fluoro, bromo, nitro, or methoxy.

14. A compound of Claim. 1 which is
5-Chloro-N-(2-hydroxyethyl)-2-(4-iodo-2-methyl-phenylamino)-benzamide;
4-Fluoro-2-(4-iodo-2-methyl-phenylamino)-benzamide;
4-Fluoro-2-(4-iodo-2-methyl-phenylamino)-N-methyl-benzamide;
N-Ethyl-4-fluoro-2-(4-iodo-2-methyl-phenylamino)-benzamide;
4-Fluoro-2-(4-iodo-2-methyl-phenylamino)-N,N-dimethyl-benzamide;
4-Fluoro-2-(4-iodo-2-methyl-phenylamino)-N-(1H-tetrazol-5-yl)-benzamide;
5-Bromo-2-(4-iodo-2-methyl-phenylamino)-benzamide;
5-Chloro-2-(4-iodo-2-methyl-phenylamino)-N,N-dimethyl-benzamide;
5-Chloro-2-(4-iodo-2-methyl-phenylamino)-N-hydroxycarbonylmethyl-benzamide;
4-Fluoro-2-(4-iodo-2-methyl-phenylamino)-N-propyl-benzamide;
5-Bromo-N-(2-hydroxy-ethyl)-2-(4-iodo-2-methyl-phenylamino)-benzamide;
N,N-Diethyl-4-fluoro-2-(4-iodo-2-methyl-phenylamino)-benzamide;
4-Fluoro-N-{3-[4-(2-hydroxy-ethyl)-piperazin-1-yl]-propyl}-2-(4-iodo-2-methyl-phenylamino)-benzamide;
N,N-Diethyl-2-(4-iodo-2-methyl-phenylamino)-5-nitro-benzamide;
N-Butyl-4-fluoro-2-(4-iodo-2-methyl-phenylamino)-benzamide;
5-Chloro-N,N-diethyl-2-(4-iodo-2-methyl-phenylamino)-benzamide;
5-Bromo-2-(4-iodo-2-methyl-phenylamino)-N,N-dimethyl-benzamide;
5-Bromo-3,4-difluoro-N-(2-hydroxy-ethyl)-2-(4-iodo-2-methyl-phenylamino)-benzamide;
N-(2,3-Dihydroxy-propyl)-3,4-difluoro-2-(4-iodo-2-methyl-phenylamino)-benzamide;
5-Bromo-3,4-difluoro-2-(4-iodo-2-methyl-phenylamino)-N-(2-piperidin-1-yl-ethyl)-benzamide;
3,4-Difluoro-N-(2-hydroxy-ethyl)-1-(4-iodo-2-methyl-phenylamino)-benzamide;
N-(2,3-Dihydroxy-propyl)-4-fluoro-2-(4-iodo-2-methyl-phenylamino)-benzamide;
3,4-Difluoro-N-(3-hydroxy-propyl)-2-(4-iodo-2-methyl-phenylamino)-benzamide;
5-Bromo-3,4-difluoro-2-(4-iodo-2-methyl-phenylamino)-N-(2-pyrrolidin-1-yl-ethyl)-benzamide;
5-Bromo-3,4-difluoro-2-(4-iodo-2-methyl-phenylamino)-N-(2-pyridin-4-yl-ethyl)-benzamide;
4-Fluoro-N-(2-hydroxy-ethyl)-2-(4-iodo-2-methyl-phenylamino)-benzamide;
5-Bromo-N-(3-dimethylamino-propyl)-3,4-difluoro-2-(4-iodo-2-methyl-phenylamino)-benzamide;
5-Bromo-3,4-difluoro-2-(4-iodo-2-methyl-phenylamino)-N-(2-morphol in-4-yl-ethyl)-benzamide;
3,4-Difluoro-2-(4-iodo-2-methyl-phenylamino)-N-(2-morpholin-4-yl-ethyl)-benzamide;
3,4-Difluoro-2-(4-iodo-2-methyl-phenylamino)-N-(2-pyrrolidin-1-yl-ethyl)-benzamide;
3,4-Difluoro-2-(4-iodo-2-methyl-phenylamino)-N-(2-pyridin-4-yl-ethyl)-benzamide;
N-(3-Dimethylamino-propyl)-3,4-difluoro-2-(4-iodo-2-methyl-phenylamino)-benzamide;
N-Benzyl-4-fluoro-2-(4-iodo-2-methyl-phenylamino)-benzamide;
2-(4-Bromo-2-methyl-phenyl amino)-3,4-difluoro-N-(2-hydroxy-ethyl)-benzamide;
4-Fluoro-2-(4-iodo-2-methyl-phenylamino)-N-(2-morpholin-4-yl-ethyl)-benzamide;
4-Fluoro-2-(4-iodo-2-methyl-phenylamino)-N-(3-piperidin-1-yl-propyl)-benzamide;
3,4-Difluoro-2-(4-iodo-2-methyl-phenylamino)-N-(3-piperidin-1-yl-propyl)-benzamide;
4-Fluoro-2-(4-iodo-2-methyl-phenylamino)-N-(2-thiophen-2-yl-ethyl)-benzamide;
4-Fluoro-2-(4-iodo-2-methyl-phenylamino)-N-(2-pyrrolidin-1-yl-ethyl)-benzamide;
2-(4-Bromo-2-methyl-phenylamino)-3,4-difluoro-N-(2-morpholin-4-yl-ethyl)-benzamide;
5-Bromo-3,4-difluoro-2-(4-iodo-2-methyl-phenylamino)-N-pyridin-4-ylmethyl-benzamide;
3,4-Difluoro-2-(4-iodo-2-methyl-phenylamino)-N-pyridin-4-ylmethyl-benzamide;
2-(4-Bromo-2-methyl-phenylamino)-N-(3-dimethylaminopropyl)-3,4-difluoro-benzamide;
4-Fluoro-2-(4-iodo-2-methyl-phenylamino)-N-pyridin-4-ylmethyl-benzamide;
4-Fluoro-2-(4-iodo-2-methyl-phenylamino)-N-(2-pyridin-4-yl-ethyl)-benzamide;
2-(4-Bromo-2-methyl-phenylamino)-3,4-difluoro-N-(2-pyridin-4-yl-ethyl)-benzamide;
2-(4-Bromo-2-methyl-phenylamino)-3,4-difluoro-N-(3-hydroxy-propyl)-benzamide;
2-(4-Bromo-2-methyl-phenylamino)-3,4-difluoro-N-(2-pyrrolidin-1-yl-ethyl)-benzamide;
4-Fluoro-2-(4-iodo-2-methyl-phenylamino)-N-phenethyl-benzamide;
2-(4-Bromo-2-methyl-phenylamino)-3,4-difluoro-N-(2-thiophen-2-yl-ethyl)-benzamide;
2-(4-Bromo-2-methyl-phenylamino)-3,4-difluoro-N-pyridin-4-ylmethyl-benzamide;
2-(4-Bromo-2-methyl-phenylamino)-3,4-difluoro-N-phenethyl-benzamide;
2-(4-Bromo-2-methyl-phenylamino)-3,4-difluoro-N-(2-piperidin-1-yl-ethyl)-benzamide;
5-Chloro-N-{3-[4-(2-hydroxy-ethyl)-piperazin-1-yl]-propyl}-2-(4-iodo-2-methyl-phenylamino)-benzamide;
5-Fluoro-N-{3-[4-(2-hydroxy-ethyl)-piperazin-1-yl]-propyl}-2-(4-iodo-2-methyl-phenylamino)-benzamide;
2-(4-Iodo-2-methyl-phenylamino)-5-nitro-N-pyridin-4-ylmethyl-benzamide;
5-Bromo-N-{3-[4-(2-hydroxy-ethyl)-piperazin-1-yl]-propyl}-2-(4-iodo-2-methyl-phenylamino)-benzamide;
5-Chloro-N-(2-diethylamino-ethyl)-2-(4-iodo-2-methyl-phenylamino)-benzamide;
5-Chloro-2-(4-iodo-2-methyl-phenylamino)-N-(2-piperidin-1-yl-ethyl)-benzamide;
(3-Hydroxy-pyrrolidin-1-yl)-[2-(4-iodo-2-methyl-phenylamino)-5-nitro-phenyl]-methanone;
5-Chloro-2-(4-iodo-2-methyl-phenylamino)-N-(2-pyrrolidin-1-yl-ethyl)-benzamide;
5-Bromo-N-(2-diethylamino-ethyl)-2-(4-iodo-2-methyl-phenylamino)-benzamide;
N-{2-[Bis-(2-hydroxy-ethyl)-amino]-ethyl}-5-chloro-2-(4-iodo-2-methylphenylamino)-benzamide;
N-{2-[Bis-(2-hydroxy-ethyl)-amino]-ethyl}-5-bromo-2-(4-iodo-2-methylphenylamino)-benzamide;
N-{3-[4-(2-Hydroxy-ethyl)-piperazin-1-yl]-propyl}-2-(4-iodo-2-methylphenylamino)-benzamide;
5-Fluoro-2-(4-iodo-2-methyl-phenylamino)-N-pyridin-4-ylmethyl-benzamide;
5-Bromo-2-(4-iodo-2-ethyl-phenylamino)-N-(2-pyrrolidin-1-yl-ethyl)-benzamide;
5-Bromo-2-(4-iodo-2-methyl-phenylamino)-N-(2-piperidin-1-yl-ethyl)-benzamide;
5-Fluoro-2-(4-iodo-2-methyl-phenylamino)-N-(2-pyrrolidin-1-yl-ethyl)-benzamide;
5-Chloro-N-(3-dimethylamino-propyl)-2-(4-iodo-2-methyl-phenylamino)-benzamide;
N-{2-[Bis-(2-hydroxy-ethyl)-amino]-ethyl}-5-fluoro-2-(4-iodo-2-methylphenylamino)-benzamide;
5-Chloro-N-(3-hydroxy-propyl)-2-(4-iodo-2-methyl-phenylamino)-benzam i d e;
5-Chloro-N-(3-diethylamino-2-hydroxy-propyl)-2-(4-iodo-2-methyl-phenylamino)-benzamide;
5-Fluoro-2-(4-iodo-2-methyl-phenylamino)-N-(2-piperidin-1-yl-ethyl)-benzamide;
5-Bromo-N-(3-hydroxy-propyl)-2-(4-iodo-2-methyl-phenylamino)-benzamide;
5-Bromo-2-(4-iodo-2-methyl-phenylamino)-N-(3-piperidin-1-yl-propyl)-benzamide;
N-{2-[Bis-(2-hydroxy-ethyl)-amino]-ethyl}-2-(4-iodo-2-methyl-phenylamino)-5-nitro-benzamide;
5-Chloro-2-(4-iodo-2-methyl-phenylamino)-N-(2-morpholin-4-yl-ethyl)-benzamide;
5-Chloro-N-(3-diethylamino-propyl)-2-(4-iodo-2-methyl-phenylamino)-benzamide;
5-Chloro-N-(2-diisopropylamino-ethyl)-2-(4-iodo-2-methyl-phenylamino)-benzamide;
5-Chloro-2-(4-iodo-2-methyl-phenylamino)-N-(3-piperidin-1-yl-propyl)-benzamide;
2-(4-Iodo-2-methyl-phenylamino)-5-nitro-N-(2-piperidin-1-yl-ethyl)-benzamide;
5-Bromo-2-(4-iodo-2-methyl-phenylamino)-N-(2-piperazin-1-yl-ethyl)-benzamide;
N-(2-Diethylamino-ethyl)-5-fluoro-2-(4-iodo-2-methyl-phenylamino)-benzamide;
5-Bromo-N-(3-dimethylamino-propyl)-2-(4-iodo-2-methyl-phenylamino)-benzamide;
N-(3-Hydroxy-propyl)-2-(4-iodo-2-methyl-phenylamino)-5-nitro-benzamide;
5-Fluoro-N-(3-hydroxy-propyl)-2-(4-iodo-2-methyl-phenylamino)-benzamide;
N-(3-Diethylamino-propyl)-5-fluoro-2-(4-iodo-2-methyl-phenylamino)-benzamide;
N-(3-Diethylamino-propyl)-2-(4-iodo-2-methyl-phenylamino)-5-nitro-benzamide;
5-Bromo-2-(4-iodo-2-methyl-phenylamino)-N-(2-morpholin-4-yl-ethyl)-benzamide;
2-(4-Iodo-2-methyl-phenylamino)-5-nitro-N-(3-piperidin-1-yl-propyl)-benzamide;
1-[5-Fluoro-2-(4-iodo-2-methyl-phenylamino)-phenyl]-1-(3-hydroxy-pyrrolidin-1-yl)-methanone;
5-Bromo-N-(2-diisopropylamino-ethyl)-2-(4-iodo-2-methyl-phenylamino)-benzamide;
5-Fluoro-2-(4-iodo-2-methyl-phenylamino)-N-(2-morpholin-4-yl-ethyl)-benzamide;
5-Fluoro-2-(4-iodo-2-methyl-phenylamino)-N-(3-piperidin-1-yl-propyl)-benzamide;
1-[5-Fluoro-2-(4-iodo-2-methyl-phenylamino)-phenyl]-1-[4-(2-hydroxy-ethyl)-piperazin-1-yl]-methanone;
N-(3-Diethylamino-2-hydroxy-propyl)-5-fluoro-2-(4-iodo-2-methylphenylamino)-benzamide;
N-Cyclopropyl-5-fluoro-2-(4-iodo-2-methyl-phenylamino)-benzamide;
5-Chloro-N-(2-hydroxy-ethyl)-2-(4-iodo-2-methyl-phenylamino)-benzamide;
5-Fluoro-N-(2-hydroxy-ethyl)-2-(4-iodo-2-methyl-phenylamino)-benzamide;
N-Benzyloxy-5-fluoro-2-(4-iodo-2-methyl-phenylamino)-benzamide;
N-Benzyloxy-5-bromo-2-(4-iodo-2-methyl-phenylamino)-benzamide;
2-(4-Iodo-2-methyl-phenylamino)-5-nitro-N-(4-sulfamoyl-benzyl)-benzamide;
5-Bromo-N-(2-hydroxy-ethyl)-2-(4-iodo-2-methyl-phenylamino)-benzamide;
N-(2-Hydroxy-ethyl)-5-iodo-2-(4-iodo-2-methyl-phenylamino)-benzamide;
N-(2-Hydroxy-ethyl)-2-(4-iodo-2-ethyl-phenylamino)-5-nitro-benzamide;
2-(4-Iodo-2-methyl-phenylamino)-N-methyl-5-nitro-N-phenyl-benzamide;
5-Chloro-N-cyclopropyl-2-(4-iodo-2-methyl-phenylamino)-benzamide;
5-Fluoro-2-(4-iodo-2-methyl-phenylamino)-N-methyl-N-phenyl-benzamide;
N-Allyl-5-fluoro-2-(4-iodo-2-methyl-phenylamino)-benzamide;
N-Benzyloxy-5-iodo-2-(4-iodo-2-methyl-phenylamino)-benzamide;
5-Fluoro-2-(4-iodo-2-methyl-phenylamino)-N-(4-sulfamoyl-benzyl)-benzamide;
N-Allyl-5-chloro-2-(4-iodo-2-methyl-phenylamino)-benzamide;
N-Cyclopropyl-2-(4-iodo-2-methyl-phenylamino)-5-nitro-benzamide;
5-Bromo-N-cyclopropyl-2-(4-iodo-2-methyl-phenylamino)-benzamide;
5-Chloro-2-(4-iodo-2-methyl-phenylamino)-N-methyl-N-phenyl-benzamide;
5-Iodo-2-(4-iodo-2-methyl-phenylamino)-N-(4-sulfamoyl-benzyl)-benzamide;
5-Bromo-2-(4-iodo-2-methyl-phenylamino)-N-(4-sulfamoyl-benzyl)-benzamide;
N-Allyl-2-(4-iodo-2-methyl-phenylamino)-5-nitro-benzamide; 2-(4-Iodo-2-methyl-phenylamino)-5-nitro-N-(4-sulfamoyl-benzyl)-benzamide;
N-Allyl-5-bromo-2-(4-iodo-2-methyl-phenylamino)-benzamide;
5-Fluoro-2-(4-iodo-2-methyl-phenylamino)-N-(3-methyl-benzyl)-benzamide;
N-Cyclopropyl-5-iodo-2-(4-iodo-2-methyl-phenylamino)-benzamide;
5-Bromo-2-(4-iodo-2-methyl-phenylamino)-N-methyl-N-phenyl-benzamide;
N-Benzyloxy-2-(4-iodo-2-methyl-phenylamino)-5-nitro-benzamide;
N-Cyclohexyl-5-iodo-2-(4-iodo-2-methyl-phenylamino)-benzamide;
N-Allyl-5-iodo-2-(4-iodo-2-methyl-phenylamino)-benzamide;
5-Iodo-2-(4-iodo-2-methyl-phenylamino)-N-(3-methyl-benzyl)-benzamide;
2-(4-Iodo-2-methyl-phenylamino)-N-(3-methyl-benzyl)-5-nitro-benzamide;
5-Iodo-2-(4-iodo-2-methyl-phenylamino)-N-methyl-N-phenyl-benzarnide;
N-Cyclohexyl-5-fluoro-2-(4-iodo-2-methyl-phenylamino)-benzamide;
5-Chloro-N-cyclohexyl-2-(4-iodo-2-methyl-phenylamino)-benzamide;
5-Bromo-2-(4-iodo-2-methyl-phenylamino)-N-(3-methyl-benzyl)-benzamide;
5-Bromo-N-cyclohexyl-2-(4-iodo-2-methyl-phenylamino)-benzamide;
5-Chloro-2-(4-iodo-2-methyl-phenylamino)-N-(3-methyl-benzyl)-benzamide;
N-Cyclohexyl-2-(4-iodo-2-methyl-phenylamino)-5-nitro-benzamide;
N-Benzyloxy-5-bromo-2-(4-iodo-2-methyl-phenylamino)-benzamide;
N-Benzyloxy-5-fluoro-2-(4-iodo-2-methyl-phenylamino)-benzamide;
5-Chloro-N-(2-hydroxy-ethyl)-2-(4-iodo-2-methyl-phenylamino)-benzamide;
5-Bromo-N-(2-hydroxy-ethyl)-2-(4-iodo-2-methyl-phenylamino)-benzamide;
2-(4-Iodo-2-methyl-phenylamino)-N-methyl-5-nitro-N-phenyl-benzamide;
5-Chloro-2-(4-iodo-2-methyl-phenylamino)-N-methyl-N-phenyl-benzamide,;
N-(2-Hydroxy-ethyl)-5-iodo-2-(4-iodo-2-methyl-phenylamino)-benzamide;
5-Chloio-N-cydopropyl-2-(4-iodo-2-methyl-phenylamino)-benzamide;
N-Allyl-5-chloro-2-(4-iodo-2-methyl-phenylamino)-benzamide;
5-Fluoro-2-(4-iodo-2-methyl-phenylamino)-N-methyl-N-phenyl-benzamide;
N-(2-Hydroxy-ethyl)-2-(4-iodo-2-methyl-phenylamino)-5-nitro-benzamide;.
5-Fluoro-N-(2-hydroxy-ethyl)-2-(4-iodo-2-methyl-phenylamino)-benzamide;
5-Bromo-N-cyclopropyl-2-(4-iodo-2-methyl-phenylamino)-benzamide;
N-Cyclopropyl-5-fluoro-2-(4-iodo-2-methyl-phenylamino)-benzamide;
5-Fluoro-2-(4-iodo-2-methyl-phenylamino)-N-(4-sulfamoyl-benzyl)-benzamide;
N-Cyclopropyl-2-(4-iodo-2-methyl-phenylamino)-5-nitro-benzamide;
N-Allyl-5-fluoro-2-(4-iodo-2-methyl-phenylamino)-benzamide;
N-Benzyloxy-5-iodo-2-(4-iodo-2-methyl-phenylamino)-benzamide;
N-Allyl-5-bromo-2-(4-iodo-2-methyl-phenylamino)-benzamide;
5-Bromo-2-(4-iodo-2-methyl-phenylamino)-N-(4-sulfamoyl-benzyl)-benzamide;
5-Bromo-2-(4-iodo-2-methyl-phenylamino)-N-methyl-N-phenyl-benzamide; and
N-Allyl-2-(4-iodo-2-methyl-phenylamino)-5-nitro-benzamide.

15. A compound of Claim 2 wherein Z is CH₂OR₇.

16. A compound of Claim 15 having the formula 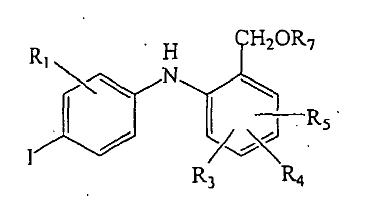 wherein:
R₁ is CH₃ or halo.

17. A compound of Claim 16 wherein: R₃ is hydrogen, chloro, or fluoro; R₄ is hydrogen, chloro, fluoro, or nitro; and R₅ is hydrogen, chloro, fluoro, bromo, nitro, or methoxy.

18. A compound of Claim 17 which is
4-Fluoro-2-(4-iodo-2-methyl-phenylamino)-benzyl alcohol;
[5-Chloro-2-(4-iodo-2-methyl-phenylamino)-phenyl]-methanol;
[2-(4-Iodo-2-methyl-phenylamino)-5-nitro-phenyl]-methanol; and
[5-Bromo-2-(4-iodo-2-methyl-phenylamino)-phenyl]-methanol.

19. A pharmaceutical formulation comprising a compound of Claim 1 together with a pharmaceutically acceptable excipient, diluent, or carrier.

20. A formulation of Claim 19 comprising a compound wherein Z is COOH or a salt thereof.

21. A formulation of Claim 19 comprising a compound wherein Z is CONR₆R₇.

22. A formulation of Claim 19 comprising a compound wherein Z is CH₂OR₇.

23. Use of a compound according to anyone of Claims 1 to 18 for preparing a pharmaceutical composition for inhibiting MEK enzymes in a mammal.

24. Use of a compound according to anyone of Claims 1 to 18 for preparing a pharmaceutical composition for treating a mammal suffering from a proliferative disease.

25. Use according to Claim 24 wherein the proliferative disease is psoriasis, restenosis, autoimmune disease, or atherosclerosis.

26. Use according to Claim 24 wherein the proliferative disease is cancer.

27. Use of a compound according to anyone of Claims 1 to 18 for preparing a pharmaceutical composition for treating a mammal suffering from stroke.

28. Use of a compound according to anyone of Claims 1 to 18 for preparing a pharmaceutical composition for treating a mammal suffering from heart failure.

29. Use of a compound according to anyone of Claims 1 to 18 for preparing a pharmaceutical composition for treatin a mammal suffering from hepatomegaly.

30. Use of a compound according to anyone of Claims 1 to 18 for preparing a pharmaceutical composition for treating a mammal suffering from cardiomegaly.

31. Use of a compound according to anyone of Claims 1 to 18 for preparing a pharmaceutical composition for treating a mammal suffering from diabetes.

32. Use of a compound according to anyone of Claims 1 to 18 for preparing a pharmaceutical composition for treating a mammal suffering from Alzheimer's disease.

33. Use of a compound according to anyone of Claims 1 to 18 for preparing a pharmaceutical composition for treating a mammal suffering from cancer.

34. Use of a compound according to anyone of Claims 1 to 18 for preparing a pharmaceutical composition for treating a mammal suffering from cystic fibrosis.

35. Use of a compound according to any one of Claims 1 to 18 for preparing a pharmaceutical composition for treating a mammal suffering from viral disease.

36. A compound which is
2,3,5-Trifluoro-4-(4-iodo-2-methyl-phenylamino)-benzoic acid;
2,3,5-Trifluoro-6-(4-iodo-2-methyl-phenylamino)-4-(4-methyl-piperazin-1-yl)-benzoic acid methyl ester dihydrofluoride salt;
5-Bromo-3,4-difluoro-2-(4-iodo-2-methyl-phenylamino)-N-(4-methyl-piperazin-1-yl)-benzamide; and
2,4-Bis-(2-chloro-4-iodo-phenylamino)-3-fluoro-5-nitro-benzoic acid.
4-Fluoro-2-(3-fluoro-4-iodo-2-methyl-phenylamino)-benzoic acid
4-Fluoro-2-(3-chloro-4-iodo-2-methyl-phenylamino)-benzoic acid
4-Fluoro2-(2,3-dimethyl-4-iodo-2 methyl-phenylamino)-benzoic acid
4-Methoxy-N-(4-methoxy-phenyl)-3-nitro benzamide

## Patentansprüche

1. Verbindung mit der Formel I 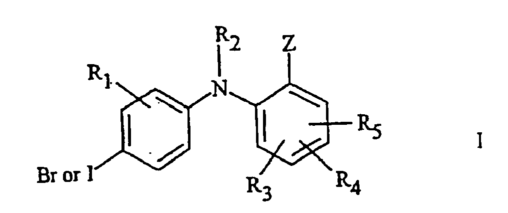 worin:
R₁ Hydroxy, C₁-C₈-Alkyl, C₁-C₈-Alkoxy, Halogen, Trifluormethyl oder CN ist;
R₂ Wasserstoff ist;
R₃, R₄ und R₅ unabhängig voneinander Wasserstoff, Hydroxy, Halogen, Trifluormethyl, C₁-C₈-Alkyl, C₁-C₈-Alkoxy, Nitro, CN oder -(O oder NH)ₘ-(CH₂)ₙ-R₉ sind, worin R₉ Wasserstoff, Hydroxy, COOH oder NR₁₀R₁₁ ist;
n 0-4 ist;
m 0 oder 1 ist;
R₁₀ und R₁₁ unabhängig voneinander Wasserstoff oder C₁-C₈₋Alkyl sind, oder zusammen mit dem Stickstoff, an den sie gebunden sind, einen 3- bis 10-gliedrigen cyclischen Ring bilden können, der gegebenenfalls 1, 2, oder 3 zusätzliche Heteroatome, ausgewählt aus 0, S, NH oder N-C₁-C₈-Alkyl enthält;
Z COOR₇, Tetrazolyl, CONR₆R₇, CONHNR₁₀R₁₁ oder CH₂OR₇ ist;
R₆ and R₇ unabhängig Wasserstoff, C₁-C₈-Alkyl, C₂-C₈₋Alkenyl, C₂-C₈-Alkinyl, 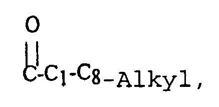 Aryl, Heteroaryl, oder C₃-C₁₀-Cycloalkyl sind, gegebenenfalls mit einem, 2 oder 3 Heteroatomen, ausgewählt aus 0, S, NH oder N-Alkyl; oder R₆ und R₇ zusammen mit dem Stickstoff, an den sie gebunden sind einen 3- bis 10-gliedrigen cyclischen Ring bilden, mit gegebenenfalls 1, 2 oder 3 zusätzlichen Heteroatomen, ausgewählt aus O, S, NH oder N-Alkyl; und worin jede der vorgenannten Alkyl-, Alkenyl- und Alkinylgruppen unsubsitutiert oder substituiert sein kann mit Halogen, Hydroxy, Alkoxy, Amino, Alkylamino, Dialkylamino, Cycloalkyl, Aryl, Aryloxy, Heteroaryl oder Heteroaryloxy; und die pharmazeutisch annehmbaren Salze davon, mit der Maßgabe, dass Verbindungen der Formel I nicht 2'-4-4'-Tribromdiphenylamin-2-Carbonsäure oder Methylesto von 2'-4-4'-Tribromdiphenylamin-2-Carbonsäure, 2[(2,4-Dibromphenyl)amino]-5-brom-benzoesäure sind.

2. Verbindung nach Anspruch 1, worin R₁ CH₃ oder Halogen ist.

3. Verbindung nach Anspruch 2, worin Z COOR₇, Tetrazolyl oder ein Salz davon ist.

4. Verbindung nach Anspruch 3 nämlich
[4-Chlor-2-(1H-tetrazol-5-yl)-phenyl]-(4-iod-2-methylphenyl)-amin;
(4-Iod-2-methyl-phenyl)-[2-(1H-tetrazol-5-yl)-phenyl]amin; und [4-Nitro-2-(1H-tetrazol-5-yl)-phenyl]-(4-iod-2-methyl-phenyl)-amin.

5. Verbindung nach Anspruch 3 der Formel

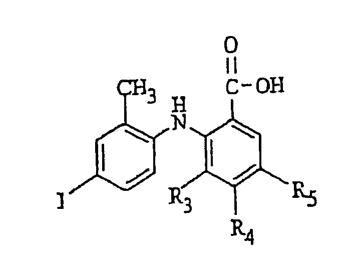

6. Verbindung nach Anspruch 5, worin R₃ Wasserstoff, Fluor oder Chlor ist, R₄ Wasserstoff, Fluor, Chlor oder Nitro ist und R₅ Wasserstoff, Chlor, Fluor, Brom, Nitro oder Methoxy ist.

7. Verbindung nach Anspruch 6 nämlich
4-Fluor-2-(4-iod-2-methylphenylamino)benzoesäure;
3,4,5-Trifluor-2-(4-iod-2-methyl-phenylamino)-benzoesäure;
3,4-Difluor-2-(4-iod-2-methyl-phenylamino)-benzoesäure;
5-Brom-3,4-difluor-2-(4-iod-2-methyl-phenylamino)-benzoesäure;
5-Chlor-2-(4-iod-2-methyl-phenylamino)-benzoesäure;
Natrium-5-chlor-2-(4-iod-2-methyl-phenylamino)-benzoate;
5-Brom-2-(4-iod-2-methyl-phenylamino)-benzoesäure;
2-(4-Iod-2-methyl-phenylamino)-5-nitro-benzoesäure;
4-Chlor-2-(4-iod-2-methyl-phenylamino)-benzoesäure;
2-(4-Iod-2-methyl-phenylamino)-benzoesäure;
5-Fluor-2-(4-iod-2-methyl-phenylamino)-benzoesäure;
2-(4-Iod-2-methyl-phenylamino)-4-nitro-benzoesäure;
und 4-Fluor-2-(4-iod-2-methyl-phenylamino)-5-nitro-benzoesäure.

8. Verbindung nach Anspruch 3 mit der Formel

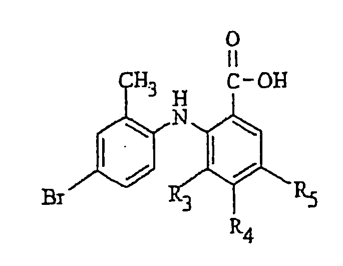

9. Verbindung nach Anspruch 8 worin R₃ Wasserstoff, Chlor oder Fluor ist; R₄ Wasserstoff, Chlor, Fluor oder Nitro ist, R₅ Wasserstoff, Chlor, Fluor, Brom, Nitro oder Methoxy ist.

10. Verbindung nach Anspruch 1 nämlich
2-(4-Brom-2-methyl-phenylamino)-4-fluor-benzoesäure;
2-(2-Brom-4-iod-phenylamino)-5-nitro-benzoesäure;
2-(4-Brom-2-methyl-phenylamino)-3,4-difluor-benzoesäure;
2-(2-Chlor-4-iod-phenylamino)-3-fluor-4-(2-morpholin-4-yl-ethylamino)-5-nitro-benzoesäure;
4-Amino-2-(2-chlor-4-iod-phenylamino)-3-fluor-5-nitro-benzoesäure;
2-(2-Chlor-4-iod-phenylamino)-4-nitro-benzoesäure;
2-(2,4-Diiod-phenylamino)-4-fluor-benzoesäure;
2-(2-Brom-4-iod-phenylamino)-4-fluor-benzoesäure;
4-Fluor-2-(2-fluor-4-iod-phenylamino)-benzoesäure;
2-(2-Chlor-4-iod-phenylamino)-4-fluor-benzoesäure;
5-Brom-2-(2-chlor-4-iod-phenylamino)-3,4-difluor-benzoesäure;
5-Iod-2-(4-iod-2-methyl-phenylamino)-benzoesäure;
2-(4-Iod-phenylamino)-5-methoxy-benzoesäure;
5-Methyl-2-(4-iod-2-methyl-phenylamino)-benzoesäure;
5-Brom-3,4-difluor-2-(4-iod-2-methyl-phenylamino)-benzoesäure-N',N'- dimethyl-hydrazid; und 4-Fluor-2-(4-iod-2-methyl-phenylamino)-benzoesäure-hydrazid.

11. Verbindung nach Anspruch 2 worin Z CONR₆R₇ ist.

12. Verbindung nach Anspruch 11 mit der Formel 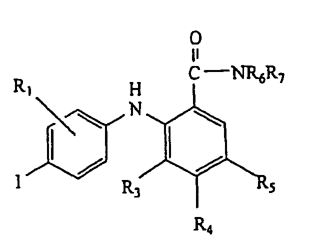 worin R₁ CH₃ oder Halogen ist.

13. Verbindung nach Anspruch 12 worin R₃ Wasserstoff, Chlor, oder Fluor ist; R₄ Wasserstoff, Chlor, Fluor, oder Nitro ist; und R₅ Wasserstoff, Chlor, Fluor, Brom, Nitro oder Methoxy ist.

14. Verbindung nach Anspruch 1 nämlich
5-Chlor-N-(2-hydroxyethyl)-2-(4-iod-2-methyl-phenylamino)-benzamid;
4-Fluor-2-(4-iod-2-methyl-phenylamino)-benzamid;
4-Fluor-2-(4-iod-2-methyl-phenylamino)-N-methyl-benzamid;
N-Ethyl-4-fluor-2-(4-iod-2-methyl-phenylamino)-benzamid;
4-Fluor-2-(4-iod-2-methyl-phenylamino)-N,N-dimethyl-benzamid;
4-Fluor-2-(4-iod-2-methyl-phenylamino)-N-(1H-tetrazol-5-yl)-benzamid;
5-Brom-2-(4-iod-2-methyl-phenylamino)-benzamid;
5-Chlor-2-(4-iod-2-methyl-phenylamino)-N,N-dimethyl-benzamid;
5-Chlor-2-(4-iod-2-methyl-phenylamino)-N-hydroxycarbonylmethyl-benzamid,
4-Fluor-2-(4-iod-2-methyl-phenylamino)-N-propyl-benzamid;
5-Brom-N-(2-hydroxy-ethyl)-2-(4-iod-2-methyl-phenylamino)-benzamid;
N,N-Diethyl-4-fluor-2-(4-iod-2-methyl-phenylamino)-benzamid;
4-Fluor-N-{3-[4-(2-hydroxy-ethyl)-piperazin-1-yl]-propyl}-2-(4-iod-2-methyl-phenylamino)-benzamid;
N,N-Diethyl-2-(4-iod-2-methyl-phenylamino)-5-nitro-benzamid;
N-Butyl-4-fluor-2-(4-iod-2-methyl-phenylamino)-benzamid;
5-Chlor-N,N-diethyl-2-(4-iod-2-methyl-phenylamino)-benzamid;
5-Brom-2-(4-iod-2-methyl-phenylamino)-N,N-dimethyl-benzamid;
5-Brom-3,4-difluor-N-(2-hydroxy-ethyl)-2-(4-iod-2-methyl-phenylamino)-benzamid;
N-(2,3-Dihydroxy-propyl)-3,4-difluor-2-(4-iod-2-methyl-phenylamino)-benzamid;
5-Brom-3,4-difluor-2-(4-iod-2-methyl-phenylamino)-N-(2-piperidin-1-yl-ethyl)-benzamid;
3,4-Difluor-N-(2-hydroxy-ethyl)-2-(4-iod-2-methyl-phenylamino)-benzamid;
N-(2,3-Dihydroxy-propyl)-4-fluor-2-(4-iod-2-methyl-phenylamino)-benzamid;
3,4-Difluor-N-(3-hydroxy-propyl)-2-(4-iod-2-methyl-phenylamino)-benzamid;
5-Brom-3,4-difluor-2-(4-iod-2-methyl-phenylamino)-N-(2-pyrrolidin-1-yl-ethyl)-benzamid;
5-Brom-3,4-difluor-2-(4-iod-2-methyl-phenylamino)-N-(2-pyridin-4-yl-ethyl)-benzamid;
4-Fluor-N-(2-hydroxy-ethyl)-2-(4-iod-2-methyl-phenylamino)-benzamid;
5-Brom-N-(3-dimethylamino-propyl)-3,4-difluor-2-(4-iod-2-methyl-phenylamnino)-benzamid;
5-Brom-3,4-difluor-2-(4-iod-2-methyl-phenylamino)-N-(2-morpholin-4-yl-ethyl)-benzamid;
3,4-Difluor-2-(4-iod-2-methyl-phenylamino)-N-(2-morpholin-4-yl-ethyl)-benzamid;
3,4-Difluor-2-(4-iod-2-methyl-phenylamino)-N-(2-pyrrolidin-1-yl-ethyl)-benzamid;
3,4-Difluor-2-(4-iod-2-methyl-phenylamino)-N-(2-pyridin-4-yl-ethyl)-benzamid;
N-(3-Dimethylamino-propyl)-3,4-difluor-2-(4-iod-2-methyl-phenylamino)-benzamid;
N-Benzyl-4-fluor-2-(4-iod-2-methyl-phenylamino)-benzamid;
2-(4-Brom-2-methyl-phenylamino)-3,4-difluor-N-(2-hydroxyethyl)-benzamid;
4-Fluor-2-(4-iod-2-methyl-phenylamino)-N-(2-morpholin-4-yl-ethyl)-benzamid;
4-Fluor-2-(4-iod-2-methyl-phenylamino)-N-(3-piperidin-1-yl-propyl)-benzamid;
3,4-Difluor-2-(4-iod-2-methyl-phenylamino)-N-(3-piperidin-1-yl-propyl)-benzamid;
4-Fluor-2-(4-iod-2-methyl-phenylamino)-N-(2-thiophen-2-yl-ethyl)-benzamid;
4-Fluor-2-(4-iod-2-methyl-phenylamino)-N-(2-pyrrolidin-1 -yl-ethyl)-benzamid;
2-(4-Brom-2-methyl-phenylamino)-3,4-difluor-N-(2-morpholin-4-yl-ethyl)-benzamid;
5-Brom-3,4-difluor-2-(4-iod-2-methyl-phenylamino)-N-pyridin-4-ylmethyl-benzamid;
3,4-Difluor-2-(4-iod-2-methyl-phenylamino)-N-pyridin-4-ylmethyl-benzamid;
2-(4-Brom-2-methyl-phenylamino)-N-(3-dimethylaminopropyl)-3,4-difluor-benzamid;
4-Fluor-2-(4-iod-2-methyl-phenylamino)-N-pyridin-4-ylmethyl-benzamid;
4-Fluor-2-(4-iod-2-methyl-phenylamino)-N-(2-pyridin-4-yl-ethyl)-benzamid;
2-(4-Brom-2-methyl-phenylamino)-3,4-difluor-N-(2-pyridin-4-yl-ethyl)-benzamid;
2-(4-Brom-2-methyl-phenylamino)-3,4-difluor-N-(3-hydroxypropyl)-benzamid;
2-(4-Brom-2-methyl-phenylamino.)-3,4-difluor-N-(2-pyrrolidin-1-yl-ethyl)-benzamid;
4-Fluor-2-(4-iod-2-methyl-phenylamino)-N-phenethyl-benzamid;
2-(4-Brom-2-methyl-phenylamino)-3,4-difluor-N-(2-thiophen-2-yl-ethyl)-benzamid;
2-(4-Brom-2-methyl-phenylamino)-3,4-difluor-N-pyridin-4-ylmethyl-benzamid;
2-(4-Brom-2-methyl-phenylamino)-3,4-difluor-N-phenethyl-benzamid;
2-(4-Brom-2-methyl-phenylamino)-3,4-difluor-N-(2-piperidin-1-yl-ethyl)-benzamid;
5-Chlor-N-{3-[4-(2-hydroxy-ethyl)-piperazin-1-yl]-propyl}-2-(4-iod-2-methyl-phenylamino)-benzamid;
5-Fluor-N-{3-[4-(2-hydroxy-ethyl)-piperazin-1-yl]-propyl}-2-(4-iod-2-methyl-phenylamino)-benzamid;
2-(4-Iod-2-methyl-phenylamino)-5-nitro-N-pyridin-4-ylmethyl-benzamid;
5-Brom-N-{3-[4-(2-hydroxy-ethyl)-piperazin-1-yl]-propyl}-2-(4-iod-2-methyl-phenylamino)-benzamid;
5-Chlor-N-(2-diethylamino-ethyl)-2-(4-iod-2-methyl-phenylamino)-benzamid;
5-Chlor-2-(4-iod-2-methyl-phenylamino)-N-(2-piperidin-1-yl-ethyl)-benzamid;
(3-Hydroxy-pyrrolidin-1-yl)-[2-(4-iod-2-methyl-phenylamino)-5-nitro-phenyl]-methanon;
5-Chlor-2-(4-iod-2-methyl-phenylamino)-N-(2-pyrrolidin-1-yl-ethyl)-benzamid;
5-Brom-N-(2-diethylamino-ethyl)-2-(4-iod-2-methyl-phenylamino)-benzamid;
N-{2-[Bis-(2-hydroxy-ethyl)-amino]-ethyl}-5-chlor-2-(4-iod-2-methyl-phenylamino)-benzamid;
N-{2-[Bis-(2-hydroxy-ethyl)-amino]-ethyl}-5-brom-2-(4-iod-2-methyl-phenylamino)-benzamid;
N-{3-[4-(2-Hydroxy-ethyl)-piperazin-1-yl]-propyl}-2-(4-iod-2-methyl-phenylamino)-benzamid;
5-Fluor-2-(4-iod-2-methyl-phenylamino)-N-pyridin-4-ylmethyl-benzamid;
5-Brom-2-(4-iod-2-ethyl-phenylamino)-N-(2-pyrrolidin-1-yl-ethyl-benzamid;
5-Brom-2-(4-iod-2-methyl-phenylamino)-N-(2-piperidin-1-yl-ethyl)-benzamid;
5-Fluor-2-(4-iod-2-methyl-phenylamino)-N-(2-pyrrolidin-1-yl-ethyl)-benzamid;
5-Chlor-N-(3-dimethylamino-propyl)-2-(4-iod-2-methyl-phenylamino)-benzamid;
N-{2-[Bis-(2-hydroxy-ethyl)-amino]-ethyl}-5-fluor-2-(4-iod-2-methyl-phenylamino)-benzamid;
5-Chlor-N-(3-hydroxy-propyl)-2-(4-iod-2-methyl-phenylamino)-benzamid;
5-Chlor-N-(3-diethylamino-2-hydroxy-propyl)-2-(4-iod-2-methyl-phenylamino)-benzamid;
5-Fluor-2-(4-iod-2-methyl-phenylamino)-N-(2-piperidin-1-yl-ethyl)-benzamid;
5-Brom-N-(3-hydroxy-propyl)-2-(4-iod-2-methyl-phenylamino)-benzamid;
5-Brom-2-(4-iod-2-methyl-phenylamino)-N-(3-piperidin-1-yl-propyl)-benzamid;
N-{2-[Bis-(2-hydroxy-ethyl)-amino]-ethyl}-2-(4-iod-2-methyl-phenylamino)-5-nitro-benzamid;
5-Chlor-2-(4-iod-2-methyl-phenylamino)-N-(2-morpholin-4-yl-ethyl)-benzamid;
5-Chlor-N-(3-diethylamino-propyl)-2-(4-iod-2-methyl-phenylamino)-benzamid;
5-Chlor-N-(2-diisopropylamino-ethyl)-2-(4-iod-2-methyl-phenylamino)-benzamid;
5-Chlor-2-(4-iod-2-methyl-phenylamino)-N-(3-piperidin-1-yl-propyl)-benzamid;
2-(4-Iod-2-methyl-phenylamino)-5-nitro-N-(2-piperidin-1-yl-ethyl)-benzamid;
5-Brom-2-(4-iod-2-methyl-phenylamino)-N-(2-piperazin-1-yl-ethyl)-benzamid;
N-(2-Diethylamino-ethyl)-5-fluor-2-(4-iod-2-methyl-phenylamino)-benzamid;
5-Brom-N-(3-dimethylamino-propyl)-2-(4-iod-2-methyl-phenylamino)-benzamid;
N-(3-Hydroxy-propyl)-2-(4-iod-2-methyl-phenylamino)-5-nitro-benzamid;
5-Fluor-N-(3-hydroxy-propyl)-2-(4-iod-2-methyl-phenylamino)-benzamid;
N-(3-Diethylamino-propyl)-5-fluor-2-(4-iod-2-methyl-phenylamino)-benzamid;
N-(3-Diethylamino-propyl)-2-(4-iod-2-methyl-phenylamino)-5-nitro-benzamid;
5-Brom-2-(4-iod-2-methyl-phenylamino)-N-(2-morpholin-4-yl-ethyl)-benzamid;
2-(4-Iod-2-methyl-phenylamino)-5-nitro-N-(3-piperidin-1-yl-propyl)-benzamid;
1-[5-Fluor-2-(4-iod-2-methyl-phenylamino)-phenyl]-1-(3-hydroxy-pyrrolidin-1-yl)-methanon;
5-Brom-N-(2-diisopropylamino-ethyl)-2-(4-iod-2-methyl-phenylamino)-benzamid;
5-Fluor-2-(4-iod-2-methyl-phenylamino)-N-(2-morpholin-4-yl-ethyl)-benzamid;
5-Fluor-2-(4-iod-2-methyl-phenylamino)-N-(3-piperidin-1-yl-propyl)-benzamid;
1-[5-Fluor-2-(4-iod-2-methyl-phenylamino)-phenyl]-1-[4-(2-hydroxy-ethyl)-piperazin-1-yl]-methanon;
N-(3-Diethylamino-2-hydroxy-propyl)-5-fluor-2-(4-iod-2-methyl-phenylamino)-benzamid;
N-Cyclopropyl-5-fluor-2-(4-iod-2-methyl-phenylamino)-benzamid;
5-Chlor-N-(2-hydroxy-ethyl)-2-(4-iod-2-methyl-phenylamino)-benzamid;
5-Fluor-N-(2-hydroxy-ethyl)-2-(4-iod-2-methyl-phenylamino)-benzamid;
N-Benzyloxy-5-fluor-2-(4-iod-2-methyl-phenylamino)-benzamid;
N-Benzyloxy-5-brom-2-(4-iod-2-methyl-phenylamino)-benzamid;
2-(4-Iod-2-methyl-phenylamino)-5-nitro-N-(4-sulfamoyl-benzyl)-benzamid;
5-Brom-N-(2-hydroxy-ethyl)-2-(4-iod-2-methyl-phenylamino)-benzamid;
N-(2-Hydroxy-ethyl)-5-iod-2-(4-iod-2-methyl-phenylamino)-benzamid;
N-(2-Hydroxy-ethyl)-2-(4-iod-2-ethyl-phenylamino)-5-nitro-benzamid;
2-(4-Iod-2-methyl-phenylamino)-N-methyl-5-nitro-N-phenyl-benzamid;
5-Chlor-N-cyclopropyl-2-(4-iod-2-methyl-phenylamino)-benzamid;
5-Fluor-2-(4-iod-2-methyl-phenylamino)-N-methyl-N-phenyl-benzamid;
N-Allyl-5-fluor-2-(4-iod-2-methyl-phenylamino)-benzamid;
N-Benzyloxy-5-iod-2-(4-iod-2-methyl-phenylamino)-benzamid;
5-Fluor-2-(4-iod-2-methyl-phenylamino)-N-(4-sulfamoyl-benzyl)-benzamid;
N-Allyl-5-chlor-2-(4-iod-2-methyl-phenylamino)-benzamid;
N-Cyclopropyl-2-(4-iod-2-methyl-phenylamino)-5-nitro-benzamid;
5-Brom-N-cyclopropyl-2-(4-iod-2-methyl-phenylamino)-benzamid;
5-Chlor-2-(4-iod-2-methyl-phenylamino)-N-methyl-N-phenyl-benzamid;
5-Iod-2-(4-iod-2-methyl-phenylamino)-N-(4-sulfamoyl-benzyl)-benzamid;
5-Brom-2-(4-iod-2-methyl-phenylamino)-N-(4-sulfamoyl-benzyl)-benzamid;
N-Allyl-2-(4-iod-2-methyl-phenylamino)-5-nitro-benzamid;
2-(4-Iod-2-methyl-phenylamino)-5-nitro-N-(4-sulfamoyl-benzyl)-benzamid;
N-Allyl-5-brom-2-(4-iod-2-methyl-phenylamino)-benzamid;
5-Fluor-2-(4-iod-2-methyl-phenylamino)-N-(3-methyl-benzyl)-benzamid;
N-Cyclopropyl-5-iod-2-(4-iod-2-methyl-phenylamino)-benzamid;
5-Brom-2-(4-iod-2-methyl-phenylamino)-N-methyl-N-phenyl-benzamid;
N-Benzyloxy-2-(4-iod-2-methyl-phenylamino)-5-nitro-benzamid;
N-Cyclohexyl-5-iod-2-(4-iod-2-methyl-phenylamino)-benzamid;
N-Allyl-5-iod-2-(4-iod-2-methyl-phenylamino)-benzamid;
5-Iod-2-(4-iod-2-methyl-phenylamino)-N-(3-methyl-benzyl)-benzamid;
2-(4-Iod-2-methyl-phenylamino)-N-(3-methyl-benzyl)-5-nitro-benzamid;
5-Iod-2-(4-iod-2-methyl-phenylamino)-N-methyl-N-phenyl-benzamid;
N-Cyclohexyl-5-fluor-2-(4-iod-2-methyl-phenylamino)-benzamid;
5-Chlor-N-cyclohexyl-2-(4-iod-2-methyl-phenylamino)-benzamid;
5-Brom-2-(4-iod-2-methyl-phenylamino)-N-(3-methylbenzyl)-benzamid;
5-Brom-N-cyclohexyl-2-(4-iod-2-methyl-phenylamino)-benzamid;
5-Chlor-2-(4-iod-2-methyl-phenylamino)-N-(3-methylbenzyl)-benzamid;
N-Cyclohexyl-2-(4-iod-2-methyl-phenylamino)-5-nitro-benzamid;
N-Benzyloxy-5-brom-2-(4-iod-2-methyl-phenylamino)-benzamid;
N-Benzyloxy-5-fluor-2-(4-iod-2-methyl-phenylamino)-benzamid;
5-Chlor-N-(2-hydroxy-ethyl)-2-(4-iod-2-methyl-phenylamino)-benzamid;
5-Brom-N-(2-hydroxy-ethyl)-2-(4-iod-2-methyl-phenylamino)-benzamid;
2-(4-Iod-2-methyl-phenylamino)-N-methyl-5-nitro-N-phenyl-benzamid;
5-Chlor-2-(4-iod-2-methyl-phenylamino)-N-methyl-N-phenyl-benzamid;
N-(2-Hydroxy-ethyl)-5-iod-2-(4-iod-2-methyl-phenylamino)-benzamid;
5-Chlor-N-cyclopropyl-2-(4-iod-2-methyl-phenylamino)-benzamid;
N-Allyl-5-chlor-2-(4-iod-2-methyl-phenylamino)-benzamid;
5-Fluor-2-(4-iod-2-methyl-phenylamino)-N-methyl-N-phenyl-benzamid;
N-(2-Hydroxy-ethyl)-2-(4-iod-2-methyl-phenylamino)-5-nitro-benzamid;
5-Fluor-N-(2-hydroxy-ethyl)-2-(4-iod-2-methyl-phenylamino)-benzamid;
5-Brom-N-cyclopropyl-2-(4-iod-2-methyl-phenylamino)-benzamid;
N-Cyclopropyl-5-fluor-2-(4-iod-2-methyl-phenylamino)-benzamid;
5-Fluor-2-(4-iod-2-methyl-phenylamino)-N-(4-sulfamoyl-benzyl)-benzamid;
N-Cyclopropyl-2-(4-iod-2-methyl-phenylamino)-5-nitro-benzamid;
N-Allyl-5-fluor-2-(4-iod-2-methyl-phenylamino)-benzamid;
N-Benzyloxy-5-iod-2-(4-iod-2-methyl-phenylamino)-benzamid;
N-Allyl-5-brom-2-(4-iod-2-methyl-phenylamino)-benzamid;
5-Brom-2-(4-iod-2-methyl-phenylamino)-N-(4-sulfamoyl-benzyl)-benzamid;
5-Brom-2-(4-iod-2-methyl-phenylamino)-N-methyl-N-phenyl-benzamid;
und
N-Allyl-2-(4-iod-2-methyl-phenylamino)-5-nitro-benzamid.

15. Verbindung nach Anspruch 2, worin Z CH₂OR₇ ist

16. Verbindung nach Anspruch 15 mit der Formel 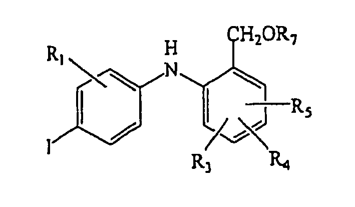 worin:
R₁ CH₃ oder Halogen ist.

17. Verbindung nach Anspruch 16, worin: R₃ Wasserstoff, Chlor, oder Fluor ist; R₄ Wasserstoff, Chlor, Fluor oder Nitro ist; und R₅ Wasserstoff, Chlor, Fluor, Brom, Nitro oder Methoxy ist.

18. Verbindung nach Anspruch 17 nämlich
4-Fluor-2-(4-iod-2-methyl-phenylamino)-benzylalkohol;
[5-Chlor-2-(4-iod-2-methyl-phenylamino)-phenyl]-methanol;
[2-(4-Iod-2-methyl-phenylamino)-5-nitro-phenyl]-methanol;
und [5-Brom-2-(4-iod-2-methyl-phenylamino)-phenyl]-methanol.

19. Pharmazeutische Formulierung, umfassend eine Verbindung nach Anspruch 1 zusammen mit einem pharmazeutisch annehmbaren Excipiens, Verdünnungsmittel oder Träger.

20. Formulierung nach Anspruch 19, umfassend eine Verbindung, worin Z COOH ist, oder ein Salz davon.

21. Formulierung nach Anspruch 19, umfassend eine Verbindung, worin Z CONR₆R₇ ist.

22. Formulierung nach Anspruch 19, umfassend eine Verbindung, worin Z CH₂OR₇ ist.

23. Verwendung einer Verbindung nach einem der Ansprüche 1 bis 18 zur Herstellung einer pharmazeutischen Zusammensetzung zur Inhibierung von MEK-Enzymen in einem Säugetier.

24. Verwendung einer Verbindung nach einem der Ansprüche 1 bis 18 zur Herstellung einer pharmazeutischen Zusammensetzung zur Behandlung eines Säugetiers, das an einer proliferativen Krankheit leidet.

25. Verwendung nach Anspruch 24, wobei die proliferative Krankheit Psoriasis, Restenose, eine Autoimmunerkrankung oder Atherosklerose ist.

26. Verwendung nach Anspruch 24, wobei die proliferative Krankheit Krebs ist.

27. Verwendung einer Verbindung nach einem der Ansprüche 1 bis 18 zur Herstellung einer pharmazeutischen Zusammensetzung zur Behandlung eines Säugetiers, das an Schlaganfall leidet.

28. Verwendung einer Verbindung nach einem der Ansprüche 1 bis 18 zur Herstellung einer pharmazeutischen Zusammensetzung zur Behandlung eines Säugetiers, das an Herzversagen leidet.

29. Verwendung einer Verbindung nach einem der Ansprüche 1 bis 18 zur Herstellung einer pharmazeutischen Zusammensetzung zur Behandlung eines Säugetiers, das an Hepatomegalie leidet.

30. Verwendung einer Verbindung nach einem der Ansprüche 1 bis 18 zur Herstellung einer pharmazeutischen Zusammensetzung zur Behandlung eines Säugetiers, das an Cardiomegalie leidet.

31. Verwendung einer Verbindung nach einem der Ansprüche 1 bis 18 zur Herstellung einer pharmazeutischen Zusammensetzung zur Behandlung eines Säugetiers, das an Diabetes leidet.

32. Verwendung n einer Verbindung nach einem der Ansprüche 1 bis 18 zur Herstellung einer pharmazeutischen Zusammensetzung zur Behandlung eines Säugetiers, das an Alzheimer leidet.

33. Verwendung einer Verbindung nach einem der Ansprüche 1 bis 18 zur Herstellung einer pharmazeutischen Zusammensetzung zur Behandlung eines Säugetiers, das an Krebs leidet.

34. Verwendung einer Verbindung nach einem der Ansprüche 1 bis 18 zur Herstellung einer pharmazeutischen Zusammensetzung zur Behandlung eines Säugetiers, das an cystischer Fibrose leidet.

35. Verwendung einer Verbindung nach einem der Ansprüche 1 bis 18 zur Herstellung einer pharmazeutischen Zusammensetzung zur Behandlung eines Säugetiers, das an viraler Erkrankung leidet.

36. Verbindung nämlich
2,3,5-Trifluor-4-(4-iod-2-methyl-phenylamino)-benzoesäure;
2,3,5-Trifluor-6-(4-iod-2-methyl-phenylamino)-4-(4-methyl-piperazin-1-yl)-benzoesäuremethylesterdihydrofluoridsalz;
5-Brom-3,4-difluor-2-(4-iod-2-methyl-phenylamino)-N-(4-methyl-piperazin-1-yl)-benzamid; und
2,4-Bis-(2-chlor-4-iod-phenylamino)-3-fluor-5-nitrobenzoesäure;
4-Fluor-2-(3-fluor-4-iod-2-methyl-phenylamino)-benzoesäure;
4-Fluor-2-(3-chlor-4-iod-2-methyl-phenylamino)-benzoesäure;
4-Fluor-2-(2,3-dimethyl-4-iod-2-methyl-phenylamino)-benzoesäure;
4-Methoxy-N-(4-methoxy-phenyl)-3-nitro-benzamid.

## Revendications

1. Composé de formule I 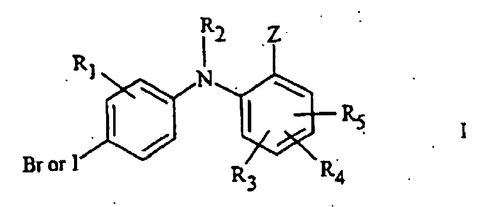 dans laquelle :
R₁ représente un groupe hydroxy, alkyle en C₁ à C₈, alkoxy en C₁ à C₈, halogéno, trifluorométhyle ou CN ;
R₂ représente un atome d'hydrogène ;
R₃, R₄ et R₅ représentent indépendamment un atome d'hydrogène, un groupe hydroxy, halogéno, trifluorométhyle, alkyle en C₁ à C₈, alkoxy en C₁ à C₈, nitro, CN ou (O ou NH)ₘ-(CH₂)ₙ-R₉ dans lequel R₉ représente un atome d'hydrogène, un groupe hydroxy, COOH ou NR₁₀R₁₁ ;
n a une valeur de 0 à 4 ;
m est égal à 0 ou 1 ;
R₁₀ et R₁₁ représentent indépendamment un atome d'hydrogène ou un groupe alkyle en C₁ à C₈, ou bien, pris conjointement avec l'atome d'azote auquel ils sont fixés, ils peuvent compléter un noyau cyclique tri- à décagonal contenant facultativement 1, 2 ou 3 hétéroatomes supplémentaires choisis entre O, S, NH ou N-(alkyle en C₁ à C₈) ;
Z représente un groupe COOR₇, tétrazolyle, CONR₆R₇, CONHNR₁₀R₁₁ ou CH₂OR₇ ;
R₆ et R₇ représentent indépendamment un atome d'hydrogène, un groupe alkyle en C₁ à C₈, alcényle en C₂ à C₈, alcynyle en C₂ à C₈, 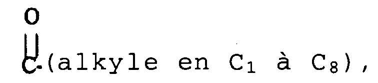 aryle, hétéroaryle ou cycloalkyle en C₃ à C₁₀ contenant facultativement un, deux ou trois hétéroatomes choisis entre O, S, NH et N-alkyle ; ou bien R₆ et R₇, conjointement avec l'atome d'azote auquel ils sont fixés, complètent un noyau cyclique tri- à décagonal contenant facultativement un, deux ou trois hétéroatomes supplémentaires choisis entre O, S, NH et N-alkyle ; n'importe lequel des groupes alkyle, alcényle et alcynyle précités pouvant être non substitués ou substitués avec des substituants halogéno, hydroxy, alkoxy, amino, alkylamino, dialkylamino, cycloalkyle, aryle, aryloxy, hétéroaryle ou hétéroaryloxy, et ses sels pharmaceutiquement acceptables, sous réserve que les composés de formule I ne soient pas l'acide 2'-4-4'-tribromodiphénylamine-2-carboxylique, l'ester méthylique d'acide 2'-4-4'-tribromodiphénylamine-2-carboxylique ou l'acide 2[(2,4-dibromophényl)amino]-5-bromo-benzoïque.

2. Composé suivant la revendication 1, dans lequel R₁ représente un groupe CH₃ ou halogéno.

3. Composé suivant la revendication 2, dans lequel Z représente un groupe COOR₇, un groupe tétrazolyle ou un de ses sels.

4. Composé suivant la revendication 3, qui est
la [4-chloro-2-(1H-tétrazole-5-yl)-phényl]-(4-iodo-2-méthyl-phényl)-amine ;
la (4-iodo-2-méthyl-phényl)-[2-(1H-tétrazole-5-yl)-phényl]-amine ; ou
la [4-nitro-2-(1H-tétrazole-5-yl)-phényl]-(4-iodo-2-méthyl-phényl)-amine.

5. Composé suivant la revendication 3, répondant à la formule

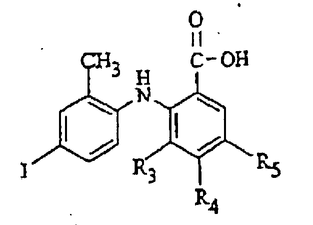

6. Composé suivant la revendication 5, dans lequel R₃ représente un atome d'hydrogène, un groupe fluoro ou chloro, R₄ représente un atome d'hydrogène, un groupe fluoro, chloro ou nitro et R₅ représente un atome d'hydrogène, un groupe chloro, fluoro, bromo, nitro ou méthoxy.

7. Composé suivant la revendication 6, qui est
l'acide 4-fluoro-2-(4-iodo-2-méthylphénylamino)benzoïque ;
l'acide 3,4,5-trifluoro-2-(4-iodo-2-méthyl-phénylamino)-benzoïque ;
l'acide 3,4-difluoro-2-(4-iodo-2-méthyl-phénylamino)-benzoïque ;
l'acide 5-bromo-3,4-difluoro-2-(4-iodo-2-méthyl-phénylamino)-benzoïque ;
l'acide 5-chloro-2-(4-iodo-2-méthyl-phénylamino)-benzoïque ;
le 5-chloro-2-(4-iodo-2-méthyl-phénylamino)-benzoate de sodium ;
l'acide 5-bromo-2-(4-iodo-2-méthyl-phénylamino)-benzoïque ;
l'acide 2-(4-iodo-2-méthyl-phénylamino)-5-nitro-benzoïque ;
l'acide 4-chloro-2-(4-iodo-2-méthyl-phénylamino)-benzoïque ;
l'acide 2-(4-iodo-2-méthyl-phénylamino)benzoïque ;
l'acide 5-fluoro-2-(4-iodo-2-méthyl-phénylamino)-benzoïque ;
l'acide 2-(4-iodo-2-méthyl-phénylamino)-4-nitro-benzoïque ; ou
l'acide 4-fluoro-2-(4-iodo-2-méthyl-phénylamino)-5-nitro-benzoïque.

8. Composé suivant la revendication 3, répondant à la formule

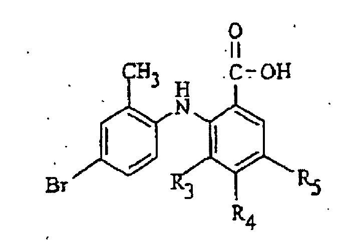

9. Composé suivant la revendication 8, dans lequel R₃ représente un atome d'hydrogène, un groupe chloro ou fluoro, R₄ représente un atome d'hydrogène, un groupe chloro, fluoro ou nitro, R₅ représente un atome d'hydrogène, un groupe chloro, fluoro, bromo, nitro ou méthoxy.

10. Composé suivant la revendication 1, qui est
l'acide 2-(4-bromo-2-méthyl-phénylamino)-4-fluoro-benzoïque ;
l'acide 2-(2-bromo-4-iodo-phénylamino)-5-nitro-benzoïque ;
l'acide 2-(4-bromo-2-méthyl-phénylamino)-3,4-difluoro-benzoïque ;
l'acide 2-(2-chloro-4-iodo-phénylamino)-3-fluoro-4-(2-morpholine-4-yl-éthylamino)-5-nitro-benzoïque ;
l'acide 4-amino-2-(2-chloro-4-iodo-phénylamino)-3-fluoro-5-nitro-benzoïque ;
l'acide 2-(2-chloro-4-iodo-phénylamino)-4-nitro-benzoïque ;
l'acide 2-(2,4-diiodo-phénylamino)-4-fluoro-benzoïque ;
l'acide 2-(2-bromo-4-iodo-phénylamino)-4-fluoro-benzoïque ;
l'acide 4-fluoro-2-(2-fluoro-4-iodo-phénylamino)-benzoïque ;
l'acide 2-(2-chloro-4-iodo-phénylamino)-4-fluoro-benzoïque ;
l'acide 5-bromo-2-(2-chloro-4-iodo-phénylamino)-3,4-difluoro-benzoïque ;
l'acide 5-iodo-2-(4-iodo-2-méthyl-phénylamino)-benzoïque ;
l'acide 2-(4-iodo-phénylamino)-5-méthoxy-benzoïque ;
l'acide 5-méthyl-2-(4-iodo-2-méthyl-phénylamino)-benzoïque;
le N',N'-diméthyl-hydrazide d'acide 5-bromo-3,4-difluoro-2-(4-iodo-2-méthyl-phénylamino)-benzoïque; ou
l'hydrazide d'acide 4-fluoro-2-(4-iodo-2-méthyl-phénylamino)-benzoïque.

11. Composé suivant la revendication 2, dans lequel Z représente un groupe CONR₆R₇.

12. Composé suivant la revendication 11, répondant à la formule 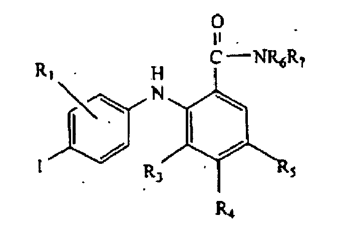 dans laquelle :
R₁ représente un groupe CH₃ ou halogéno.

13. Composé suivant la revendication 12, dans lequel R₃ représente un atome d'hydrogène, un groupe chloro ou fluoro, R₄ représente un atome d'hydrogène, un groupe chloro, fluoro ou nitro et R₅ représente un atome d'hydrogène, un groupe chloro, fluoro, bromo, nitro ou méthoxy.

14. Composé suivant la revendication 1, qui est :
le 5-chloro-N-(2-hydroxyéthyl)-2-(4-iodo-2-méthyl-phénylamino)-benzamide ;
le 4-fluoro-2-(4-iodo-2-méthyl-phénylamino)-benzamide ;
le 4-fluoro-2-(4-iodo-2-méthyl-phénylamino)-N-méthyl-benzamide ;
le N-éthyl-4-fluoro-2-(4-iodo-2-méthyl-phénylamino)-benzamide ;
le 4-fluoro-2-(4-iodo-2-méthyl-phénylamino)-N,N-diméthyl-benzamide ;
le 4-fluoro-2-(4-iodo-2-méthyl-phénylamino)-N-(1H-tétrazole-5-yl)-benzamide ;
le 5-bromo-2-(4-iodo-2-méthyl-phénylamino)-benzamide;
le 5-chloro-2-(4-iodo-2-méthyl-phénylamino)-N,N-diméthyl-benzamide ;
le 5-chloro-2-(4-iodo-2-méthyl-phénylamino)-N-hydroxy-carbonylméthyl-benzamide ;
le 4-fluoro-2-(4-iodo-2-méthyl-phénylamino)-N-propyl-benzamide ;
le 5-bromo-N-(2-hydroxy-éthyl)-2-(4-iodo-2-méthyl-phénylamino)-benzamide ;
le N,N-diéthyl-4-fluoro-2-(4-iodo-2-méthyl-phénylamino)-benzamide ;
le 4-fluoro-N-{3-[4-(2-hydroxy-éthyl)-pipérazine-1-yl]-propyl}-2-(4-iodo-2-méthyl-phénylamino)-benzamide ;
le N,N-diéthyl-2-(4-iodo-2-méthyl-phénylamino)-5-nitro-benzamide ;
le N-butyl-4-fluoro-2-(4-iodo-2-méthyl-phénylamino)-benzamide ;
le 5-chloro-N,N-diéthyl-2-(4-iodo-2-méthyl-phénylamino)-benzamide ;
le 5-bromo-2-(4-iodo-2-méthyl-phénylamino)-N,N-diméthyl-benzamide ;
le 5-bromo-3,4-difluoro-N-(2-hydroxy-éthyl)-2-(4-iodo-2-méthyl-phénylamino)-benzamide ;
le N-(2,3-dihydroxy-propyl)-3,4-difluoro-2-(4-iodo-2-méthyl-phénylamino)-benzamide ;
le 5-bromo-3,4-difluoro-2-(4-iodo-2-méthyl-phénylamino)-N-(2-pipéridine-1-yl-éthyl)-benzamide ;
le 3,4-difluoro-N-(2-hydroxy-éthyl)-2-(4-iodo-2-méthyl-phénylamino)-benzamide ;
le N-(2,3-dihydroxy-propyl)-4-fluoro-2-(4-iodo-2-méthyl-phénylamino)-benzamide ;
le 3,4-difluoro-N-(3-hydroxy-propyl)-2-(4-iodo-2-méthyl-phénylamino)-benzamide ;
le 5-bromo-3,4-difluoro-2-(4-iodo-2-méthyl-phénylamino)-N-(2-pyrrolidine-1-yl-éthyl)-benzamide ;
le 5-bromo-3,4-difluoro-2-(4-iodo-2-méthyl-phénylamino)-N-(2-pyridine-4-yl-éthyl)-benzamide ;
le 4-fluoro-N-(2-hydroxy-éthyl)-2-(4-iodo-2-méthyl-phénylamino)-benzamide ;
le 5-bromo-N-(3-diméthylamino-propyl)-3,4-difluoro-2-(4-iodo-2-méthyl-phénylamino)-benzamide ;
le 5-bromo-3,4-difluoro-2-(4-iodo-2-méthyl-phénylamino)-N-(2-morpholine-4-yl-éthyl)-benzamide ;
le 3,4-difluoro-2-(4-iodo-2-méthyl-phénylamino)-N-(2-morpholine-4-yl-éthyl)-benzamide ;
le 3,4-difluoro-2-(4-iodo-2-méthyl-phénylamino)-N-(2-pyrrolidine-1-yl-éthyl)-benzamide ;
le 3,4-difluoro-2-(4-iodo-2-méthyl-phénylamino)-N-(2-pyridine-4-yl-éthyl)-benzamide ;
le N-(3-diméthylamino-propyl)-3,4-difluoro-2-(4-iodo-2-méthyl-phénylamino)-benzamide ;
le N-benzyl-4-fluoro-2-(4-iodo-2-méthyl-phénylamino)-benzamide ;
le 2-(4-bromo-2-méthyl-phénylamino)-3,4-difluoro-N-(2-hydroxy-éthyl)-benzamide ;
le 4-fluoro-2-(4-iodo-2-méthyl-phénylamino)-N-(2-morpholine-4-yl-éthyl)-benzamide ;
le 4-fluoro-2-(4-iodo-2-méthyl-phénylamino)-N-(3-pipéridine-1-yl-propyl)-benzamide ;
le 3,4-difluoro-2-(4-iodo-2-méthyl-phénylamino)-N-(3-pipéridine-1-yl-propyl)-benzamide ;
le 4-fluoro-2-(4-iodo-2-méthyl-phénylamino)-N-(2-thiophène-2-yl-éthyl)-benzamide ;
le 4-fluoro-2-(4-iodo-2-méthyl-phénylamino)-N-(2-pyrrolidine-1-yl-éthyl)-benzamide ;
le 2-(4-bromo-2-méthyl-phénylamino)-3,4-difluoro-N-(2-morpholine-4-yl-éthyl)-benzamide ;
le 5-bromo-3,4-difluoro-2-(4-iodo-2-méthyl-phénylamino)-N-pyridine-4-ylméthyl-benzamide ;
le 3,4-difluoro-2-(4-iodo-2-méthyl-phénylamino)-N-pyridine-4-ylméthyl-benzamide ;
le 2-(4-bromo-2-méthyl-phénylamino)-N-(3-diméthylamino-propyl)-3,4-difluoro-benzamide ;
le 4-fluoro-2-(4-iodo-2-méthyl-phénylamino)-N-pyridine-4-ylméthyl-benzamide ;
le 4-fluoro-2-(4-iodo-2-méthyl-phénylamino)-N-(2-pyridine-4-yl-éthyl)-benzamide ;
le 2-(4-bromo-2-méthyl-phénylamino)-3,4-difluoro-N-(2-pyridine-4-yl-éthyl)-benzamide ;
le 2-(4-bromo-2-méthyl-phénylamino)-3,4-difluoro-N-(3-hydoxy-propyl)-benzamide ;
le 2-(4-bromo-2-méthyl-phénylamino)-3,4-difluoro-N-(2-pyrrolidine-1-yl-éthyl)-benzamide ;
le 4-fluoro-2-(4-iodo-2-méthyl-phénylamino)-N-phénéthyl-benzamide ;
le 2-(4-bromo-2-méthyl-phénylamino)-3,4-difluoro-N-(2-thiophène-2-yl-éthyl)-benzamide ;
le 2-(4-bromo-2-méthyl-phénylamino)-3,4-difluoro-N-pyridine-4-ylméthyl-benzamide ;
le 2-(4-bromo-2-méthyl-phénylamino)-3,4-difluoro-N-phénéthyl-benzamide ;
le 2-(4-bromo-2-méthyl-phénylamino)-3,4-difluoro-N-(2-pipéridine-1-yl-éthyl)-benzamide ;
le 5-chloro-N-{3-[4-(2-hydroxy-éthyl)-pipérazine-1-yl]-propyl}-2-(4-iodo-2-méthyl-phénylamino)-benzamide ;
le 5-fluoro-N-{3-[4-(2-hydroxy-éthyl)-pipérazine-1-yl]-propyl}-2-(4-iodo-2-méthyl-phénylamino)-benzamide ;
le 2-(4-iodo-2-méthyl-phénylamino)-5-nitro-N-pyridine-4-ylméthyl-benzamide ;
le 5-bromo-N-{3-[4-(2-hydroxy-éthyl)-pipérazine-1-yl]-propyl}-2-(4-iodo-2-méthyl-phénylamino)-benzamide ;
le 5-chloro-N-(2-diéthylamino-éthyl)-2-(4-iodo-2-méthyl-phénylamino)-benzamide ;
le 5-chloro-2-(4-iodo-2-méthyl-phénylamino)-N-(2-pipéridine-1-yl-éthyl)-benzamide ;
la (3-hydroxy-pyrrolidine-1-yl)-[2-(4-iodo-2-méthyl-phénylamino)-5-nitro-phényl]-méthanone ;
le 5-chloro-2-(4-iodo-2-méthyl-phénylamino)-N-(2-pyrrolidine-1-yl-éthyl)-benzamide ;
le 5-bromo-N-(2-diéthylamino-éthyl)-2-(4-iodo-2-méthyl-phénylamino)-benzamide ;
le N-{2-[bis-(2-hydroxy-éthyl)-amino]-éthyl}-5-chloro-2-(4-iodo-2-méthyl-phénylamino)-benzamide ;
le N-{2-[bis-(2-hydroxy-éthyl)-amino]-éthyl}-5-bromo-2-(4-iodo-2-méthyl-phénylamino)-benzamide ;
le N-{3-[4-(2-hydroxy-éthyl)-pipérazine-1-yl]-propyl}-2-(4-iodo-2-méthyl-phénylamino)-benzamide ;
le 5-fluoro-2-(4-iodo-2-méthyl-phénylamino)-N-pyridine-4-ylméthyl-benzamide ;
le 5-bromo-2-(4-iodo-2-éthyl-phénylamino)-N-(2-pyrrolidine-1-yl-éthyl)-benzamide ;
le 5-bromo-2-(4-iodo-2-méthyl-phénylamino)-N-(2-pipéridine-1-yl-éthyl)-benzamide ;
le 5-fluoro-2-(4-iodo-2-méthyl-phénylamino)-N-(2-pyrrolidine-1-yl-éthyl)-benzamide ;
le 5-chloro-N-(3-diméthylamino-propyl)-2-(4-iodo-2-méthyl-phénylamino)-benzamide ;
le N-{2-[bis-(2-hydroxy-éthyl)-amino]-éthyl}-5-fluoro-2-(4-iodo-2-méthyl-phénylamino)-benzamide ;
le 5-chloro-N-(3-hydroxy-propyl)-2-(4-iodo-2-méthyl-phénylamino)-benzamide ;
le 5-chloro-N-(3-diéthylamino-2-hydroxy-propyl)-2-(4-iodo-2-méthyl-phénylamino)-benzamide ;
le 5-fluoro-2-(4-iodo-2-méthyl-phénylamino)-N-(2-pipéridine-1-yl-éthyl)-benzamide ;
le 5-bromo-N-(3-hydroxy-propyl)-2-(4-iodo-2-méthyl-phénylamino)-benzamide ;
le 5-bromo-2-(4-iodo-2-méthyl-phénylamino)-N-(3-pipéridine-1-yl-propyl)-benzamide ;
le N-{2-[bis-(2-hydroxy-éthyl)-amino]-éthyl}-2-(4-iodo-2-méthyl-phénylamino)-5-nitro-benzamide ;
le 5-chloro-2-(4-iodo-2-méthyl-phénylamino)-N-(2-morpholine-4-yl-éthyl)-benzamide ;
le 5-chloro-N-(3-diéthylamino-propyl)-2-(4-iodo-2-méthyl-phénylamino)-benzamide ;
le 5-chloro-N-(2-diisopropylamino-éthyl)-2-(4-iodo-2-méthyl-phénylamino)-benzamide ;
le 5-chloro-2-(4-iodo-2-méthyl-phénylamino)-N-(3-pipéridine-1-yl-propyl)-benzamide ;
le 2-(4-iodo-2-méthyl-phénylamino)-5-nitro-N-(2-pipéridine-1-yl-éthyl)-benzamide ;
le 5-bromo-2-(4-iodo-2-méthyl-phénylamino)-N-(2-pipérazine-1-yl-éthyl)-benzamide ;
le N-(2-diéthylamino-éthyl)-5-fluoro-2-(4-iodo-2-méthyl-phénylamino)-benzamide ;
le 5-bromo-N-(3-diméthylamino-propyl)-2-(4-iodo-2-méthyl-phénylamino)-benzamide ;
le N-(3-hydroxy-propyl)-2-(4-iodo-2-méthyl-phénylamino)-5-nitro-benzamide ;
le 5-fluoro-N-(3-hydroxy-propyl)-2-(4-iodo-2-méthyl-phénylamino)-benzamide ;
le N-(3-diéthylamino-propyl)-5-fluoro-2-(4-iodo-2-méthyl-phénylamino)-benzamide ;
le N-(3-diéthylamino-propyl)-2-(4-iodo-2-méthyl-phénylamino)-5-nitro-benzamide ;
le 5-bromo-2-(4-iodo-2-méthyl-phénylamino)-N-(2-morpholine-4-yl-éthyl)-benzamide ;
le 2-(4-iodo-2-méthyl-phénylamino)-5-nitro-N-(3-pipéridine-1-yl-propyl)-benzamide ;
la 1-[5-fluoro-2-(4-iodo-2-méthyl-phénylamino)-phényl]-1-(3-hydroxy-pyrrolidine-1-yl)-méthanone ;
le 5-bromo-N-(2-diisopropylamino-éthyl)-2-(4-iodo-2-méthyl-phénylamino)-benzamide ;
le 5-fluoro-2-(4-iodo-2-méthyl-phénylamino)-N-(2-morpholine-4-yl-éthyl)-benzamide ;
le 5-fluoro-2-(4-iodo-2-méthyl-phénylamino)-N-(3-pipéridine-1-yl-propyl)-benzamide ;
la 1-[5-fluoro-2-(4-iodo-2-méthyl-phénylamino)-phényl]-1-[4-(2-hydroxy-éthyl)-pipérazine-1-yl]-méthanone ;
le N-(3-diéthylamino-2-hydroxy-propyl)-5-fluoro-2-(4-iodo-2-méthyl-phénylamino)-benzamide ;
le N-cyclopropyl-5-fluoro-2-(4-iodo-2-méthyl-phénylamino)-benzamide ;
le 5-chloro-N-(2-hydroxy-éthyl)-2-(4-iodo-2-méthyl-phénylamino)-benzamide ;
le 5-fluoro-N-(2-hydroxy-éthyl)-2-(4-iodo-2-méthyl-phénylamino)-benzamide ;
le N-benzyloxy-5-fluoro-2-(4-iodo-2-méthyl-phénylamino)-benzamide ;
le N-benzyloxy-5-bromo-2-(4-iodo-2-méthyl-phénylamino)-benzamide ;
le 2-(4-iodo-2-méthyl-phénylamino)-5-nitro-N-(4-sulfamoyl-benzyl)-benzamide ;
le 5-bromo-N-(2-hydroxy-éthyl)-2-(4-iodo-2-méthyl-phénylamino)-benzamide ;
le N-(2-hydroxy-éthyl)-5-iodo-2-(4-iodo-2-méthyl-phénylamino)-benzamide ;
le N-(2-hydroxy-éthyl)-2-(4-iodo-2-éthyl-phénylamino)-5-nitro-benzamide ;
le 2-(4-iodo-2-méthyl-phénylamino)-N-méthyl-5-nitro-N-phényl-benzamide ;
le 5-chloro-N-cyclopropyl-2-(4-iodo-2-méthyl-phénylamino)-benzamide ;
le 5-fluoro-2-(4-iodo-2-méthyl-phénylamino)-N-méthyl-N-phényl-benzamide ;
le N-allyl-5-fluoro-2-(4-iodo-2-méthyl-phénylamino)-benzamide ;
le N-benzyloxy-5-iodo-2-(4-iodo-2-méthyl-phénylamino)-benzamide ;
le 5-fluoro-2-(4-iodo-2-méthyl-phénylamino)-N-(4-sulfamoyl-benzyl)-benzamide ;
le N-allyl-5-chloro-2-(4-iodo-2-méthyl-phénylamino)-benzamide ;
le N-cyclopropyl-2-(4-iodo-2-méthyl-phénylamino)-5-nitro-benzamide ;
le 5-bromo-N-cyclopropyl-2-(4-iodo-2-méthyl-phénylamino)-benzamide ;
le 5-chloro-2-(4-iodo-2-méthyl-phénylamino)-N-méthyl-N-phényl-benzamide;
le 5-iodo-2-(4-iodo-2-méthyl-phénylamino)-N-(4-sulfamoyl-benzyl)-benzamide ;
le 5-bromo-2-(4-iodo-2-méthyl-phénylamino)-N-(4-sulfamoyl-benzyl)-benzamide ;
le N-allyl-2-(4-iodo-2-méthyl-phénylamino)-5-nitro-benzamide;
le 2-(4-iodo-2-méthyl-phénylamino)-5-nitro-N-(4-sulfamoyl-benzyl)-benzamide ;
le N-allyl-5-bromo-2-(4-iodo-2-méthyl-phénylamino)-benzamide ;
le 5-fluoro-2-(4-iodo-2-méthyl-phénylamino)-N-(3-méthyl-benzyl)-benzamide ;
le N-cyclopropyl-5-iodo-2-(4-iodo-2-méthyl-phénylamino)-benzamide ;
le 5-bromo-2-(4-iodo-2-méthyl-phénylamino)-N-méthyl-N-phényl-benzamide ;
le N-benzyloxy-2-(4-iodo-2-méthyl-phénylamino)-5-nitro-benzamide ;
le N-cyclohexyl-5-iodo-2-(4-iodo-2-méthyl-phénylamino)-benzamide ;
le N-allyl-5-iodo-2-(4-iodo-2-méthyl-phénylamino)-benzamide ;
le 5-iodo-2-(4-iodo-2-méthyl-phénylamino)-N-(3-méthyl-benzyl)-benzamide ;
le 2-(4-iodo-2-méthyl-phénylamino)-N-(3-méthyl-benzyl)-5-nitro-benzamide ;
le 5-iodo-2-(4-iodo-2-méthyl-phénylamino)-N-méthyl-N-phényl-benzamide ;
le N-cyclohexyl-5-fluoro-2-(4-iodo-2-méthyl-phénylamino)-benzamide ;
le 5-chloro-N-cyclohexyl-2-(4-iodo-2-méthyl-phénylamino)-benzamide ;
le 5-bromo-2-(4-iodo-2-méthyl-phénylamino)-N-(3-méthyl-benzyl)-benzamide ;
le 5-bromo-N-cyclohexyl-2-(4-iodo-2-méthyl-phénylamino)-benzamide ;
le 5-chloro-2-(4-iodo-2-méthyl-phénylamino)-N-(3-méthyl-benzyl)-benzamide ;
le N-cyclohexyl-2-(4-iodo-2-méthyl-phénylamino)-5-nitro-benzamide ;
le N-benzyloxy-5-bromo-2-(4-iodo-2-méthyl-phénylamino)-benzamide ;
le N-benzyloxy-5-fluoro-2-(4-iodo-2-méthyl-phénylamino)-benzamide ;
le 5-chloro-N-(2-hydroxy-éthyl)-2-(4-iodo-2-méthyl-phénylamino)-benzamide ;
le 5-bromo-N-(2-hydroxy-éthyl)-2-(4-iodo-2-méthyl-phénylamino)-benzamide ;
le 2-(4-iodo-2-méthyl-phénylamino)-N-méthyl-5-nitro-N-phényl-benzamide ;
le 5-chloro-2-(4-iodo-2-méthyl-phénylamino)-N-méthyl-N-phényl-benzamide ;
le N-(2-hydroxy-éthyl)-5-iodo-2-(4-iodo-2-méthyl-phénylamino)-benzamide ;
le 5-chloro-N-cyclopropyl-2-(4-iodo-2-méthyl-phénylamino)-benzamide ;
le N-allyl-5-chloro-2-(4-iodo-2-méthyl-phénylamino)-benzamide ;
le 5-fluoro-2-(4-iodo-2-méthyl-phénylamino)-N-méthyl-N-phényl-benzamide ;
le N-(2-hydroxy-éthyl)-2-(4-iodo-2-méthyl-phénylamino)-5-nitro-benzamide ;
le 5-fluoro-N-(2-hydroxy-éthyl)-2-(4-iodo-2-méthyl-phénylamino)-benzamide ;
le 5-bromo-N-cyclopropyl-2-(4-iodo-2-méthyl-phénylamino)-benzamide ;
le N-cyclopropyl-5-fluoro-2-(4-iodo-2-méthyl-phénylamino)-benzamide ;
le 5-fluoro-2-(4-iodo-2-méthyl-phénylamino)-N-(4-sulfamoyl-benzyl)-benzamide ;
le N-cyclopropyl-2-(4-iodo-2-méthyl-phénylamino)-5-nitro-benzamide ;
le N-allyl-5-fluoro-2-(4-iodo-2-méthyl-phénylamino)-benzamide ;
le N-benzyloxy-5-iodo-2-(4-iodo-2-méthyl-phénylamino)-benzamide ;
le N-allyl-5-bromo-2-(4-iodo-2-méthyl-phénylamino)-benzamide ;
le 5-bromo-2-(4-iodo-2-méthyl-phénylamino)-N-(4-sulfamoyl-benzyl)-benzamide ;
le 5-bromo-2-(4-iodo-2-méthyl-phénylamino)-N-méthyl-N-phényl-benzamide ;
ou
le N-allyl-2-(4-iodo-2-méthyl-phénylamino)-5-nitro-benzamide.

15. Composé suivant la revendication 2, dans lequel Z représente un groupe CH₂OR₇.

16. Composé suivant la revendication 15, répondant à la formule 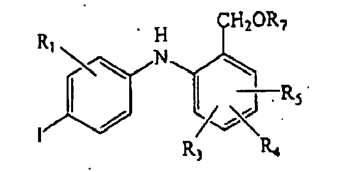 dans laquelle :
R₁ représente un groupe CH₃ ou halogéno.

17. Composé suivant la revendication 16, dans lequel :
R₃ représente un atome d'hydrogène, un groupe chloro ou fluoro; R₄ représente un atome d'hydrogène, un groupe chloro, fluoro ou nitro et R₅ représente un atome d'hydrogène, un groupe chloro, fluoro, bromo, nitro ou méthoxy.

18. Composé suivant la revendication 17, qui est
l'alcool 4-fluoro-2-(4-iodo-2-méthyl-phénylamino)-benzylique ;
le [5-chloro-2-(4-iodo-2-méthyl-phénylamino)-phényl]-méthanol ;
le [2-(4-iodo-2-méthyl-phénylamino)-5-nitro-phényl]-méthanol ; et
le [5-bromo-2-(4-iodo-2-méthyl-phénylamino)-phényl]-méthanol.

19. Formulation pharmaceutique comprenant un composé de la revendication 1 conjointement avec un excipient, diluant ou support pharmaceutiquement acceptable.

20. Formulation suivant la revendication 19, comprenant un composé dans lequel Z représente un groupe COOH ou un de ses sels.

21. Formulation suivant la revendication 19, comprenant un composé dans lequel Z représente un groupe CONR₆R₇.

22. Formulation suivant la revendication 19, comprenant un composé dans lequel Z représente un groupe CH₂OR₇.

23. Utilisation d'un composé suivant l'une quelconque des revendications 1 à 18 pour la préparation d'une composition pharmaceutique destinée à inhiber des enzymes MEK chez un mammifère.

24. Utilisation d'un composé suivant l'une quelconque des revendications 1 à 18 pour la préparation d'une composition pharmaceutique destinée au traitement d'un mammifère souffrant d'un maladie proliférative.

25. Utilisation suivant la revendication 24, dans laquelle la maladie proliférative est le psoriasis, une resténose, une maladie auto-immune ou l'athérosclérose.

26. Utilisation suivant la revendication 24, dans laquelle la maladie proliférative est le cancer.

27. Utilisation d'un composé suivant l'une quelconque des revendications 1 à 18, pour la préparation d'une composition pharmaceutique destinée au traitement d'un mammifère souffrant d'un ictus.

28. Utilisation d'un composé suivant l'une quelconque des revendications 1 à 18, pour la préparation d'une composition pharmaceutique destinée au traitement d'un mammifère souffrant d'insuffisance cardiaque.

29. Utilisation d'un composé suivant l'une quelconque des revendications 1 à 18, pour la préparation d'une composition pharmaceutique destinée au traitement d'un mammifère souffrant d'hépatomégalie.

30. Utilisation d'un composé suivant l'une quelconque des revendications 1 à 18, pour la préparation d'une composition pharmaceutique destinée au traitement d'un mammifère souffrant de cardiomégalie.

31. Utilisation d'un composé suivant l'une quelconque des revendications 1 à 18, pour la préparation d'une composition pharmaceutique destinée au traitement d'un mammifère souffrant de diabète.

32. Utilisation d'un composé suivant l'une quelconque des revendications 1 à 18, pour la préparation d'une composition pharmaceutique destinée au traitement d'un mammifère souffrant de la maladie d'Alzheimer.

33. Utilisation d'un composé suivant l'une quelconque des revendications 1 à 18, pour la préparation d'une composition pharmaceutique destinée au traitement d'un mammifère souffrant d'un cancer.

34. Utilisation d'un composé suivant l'une quelconque des revendications 1 à 18, pour la préparation d'une composition pharmaceutique destinée au traitement d'un mammifère souffrant de fibrose kystique.

35. Utilisation d'un composé suivant l'une quelconque des revendications 1 à 18, pour la préparation d'une composition pharmaceutique destinée au traitement d'un mammifère souffrant d'une maladie virale.

36. Composé qui est
l'acide 2,3,5-trifluoro-4-(4-iodo-2-méthyl-phénylamino)-benzoïque ;
le difluorhydrate d'ester méthylique d'acide 2,3,5-trifluoro-6-(4-iodo-2-méthyl-phénylamino)-4-(4-méthyl-pipérazine-1-yl)-benzoïque ;
le 5-bromo-3,4-difluoro-2-(4-iodo-2-méthyl-phénylamino)-N-(4-méthyl-pipérazine-1-yl)-benzamide ;
l'acide 2,4-bis-(2-chloro-2-iodo-phénylamino)-3-fluoro-5-nitro-benzoïque ;
l'acide 4-fluoro-2-(3-fluoro-4-iodo-2-méthyl-phénylamino)-benzoïque ;
l'acide 4-fluoro-2-(3-chloro-4-iodo-2-méthyl-phénylamino)-benzoïque ;
l'acide 4-fluoro-2-(3-fluoro-4-iodo-2-méthyl-phénylamino)-benzoïque ; ou
le 4-méthoxy-N-(4-méthoxy-phényl)-3-nitro-benzamide.
